(19) **Europäisches Patentamt**
**European Patent Office**
**Office européen des brevets**

(11) **EP 4 603 836 A1**

(12) **EUROPEAN PATENT APPLICATION**

(43) Date of publication:
20.08.2025 Bulletin 2025/34

(21) Application number: 24157256.9

(22) Date of filing: 13.02.2024

(51) International Patent Classification (IPC):
*G01N 33/574* (2006.01)

(52) Cooperative Patent Classification (CPC):
**G01N 33/57496; G01N 33/57434;** G01N 2800/52

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Koninklijke Philips N.V.**
**5656 AG Eindhoven (NL)**
• **The University Court of the University of Glasgow**
**Glasgow G12 8QQ (GB)**

(72) Inventors:
• **HOFFMANN, Ralf Dieter**
**Eindhoven (NL)**
• **EDWARDS, Joanne**
**Eindhoven (NL)**
• **BAILLIE, George**
**5656AG Eindhoven (NL)**

(74) Representative: **Philips Intellectual Property & Standards**
**High Tech Campus 52**
**5656 AG Eindhoven (NL)**

Remarks:
The complete document including Reference Table(s) and the Sequence Listing(s) can be downloaded from the EPO website

(54) **PREDICTION OF AN OUTCOME OF A PROSTATE CANCER SUBJECT**

(57) The invention relates to methods for predicting an outcome for a patient with hormone sensitive prostate cancer. In particular the methods are based on quantifying nuclear PDE4D7 protein, wherein low nuclear presence of PDE4D7 indicates a poor expected outcome and high nuclear presence of PDE4D7 status indicates a good expected outcome.

Fig. 6

**Description**

FIELD OF THE INVENTION

The invention relates to methods for predicting an outcome for a patient with hormone sensitive prostate cancer. In particular the methods are based on quantifying nuclear PDE4D7 protein, wherein low nuclear presence of PDE4D7 indicates a poor expected outcome and high nuclear presence of PDE4D7 status indicates a good expected outcome. The invention further describes an apparatus and a computer program product for performing the methods. Further described are therapies for use in the treatment, alleviation or prevention of hormone sensitive prostate cancer in a patient, the use comprising predicting an outcome for the patient based on a quantification of PDE4D7 protein in the nuclei of cells in a sample obtained from the patient. Also describes is a kit of parts comprising a PDE4D7 specific antibody and uses thereof.

INTRODUCTION

In prostate cancer cell lines and xenografts, a significant downregulation of PDE4D7 expression is observed in androgen-insensitive phenotypes compared to androgen-sensitive cells. Increased cell proliferation was reported in VCaP cells transfected with an inactive dominant negative form of PDE4D7 or treated with siRNA targeting PDE4D7. Similarly, overexpression of PDE4D7 in PC3 cells was significantly associated with decreased proliferation, suggesting that PDE4D7 suppression could drive the androgen-insensitive phenotype and sustain the growth of androgen-insensitive tumors.

Additional evidence supporting the role of PDE4D7 in prostate cancer progression includes a positive correlation between PDE4D7 mRNA expression and primary tumor development. In a cohort of 1405 patients, PDE4D7 was significantly downregulated in castrate-resistant prostate cancer (Böttcher et al., 2015). PDE4D7 correlated with low-grade disease and reduced progression after primary treatment in primary TMPRSS2-ERG-positive tumors. This led to the suggestion of PDE4D7 as a novel biomarker associated with the development and progression of prostate cancer. Furthermore, PDE4D7 mRNA expression was found to be upregulated in primary prostate cancer compared to normal adjacent prostate tissue, with a decrease as the disease progressed.

In a surgical resection tissue cohort, PDE4D7 mRNA expression significantly correlated with time to biochemical relapse, with high PDE4D7 scores indicating a low risk of postsurgical progression of the disease. Additionally, in three independent presurgical cohorts, the PDE4D7 score was associated with time to biochemical relapse and improved the well-established presurgical risk score CAPRA. The combination of CAPRA and PDE4D7 risk score, including other PDE4D long isoforms, further enhanced predictive accuracy, associating with prostate cancer progression after radical prostatectomy and adverse post-surgical pathology features. The potential translation of PDE4D7 as a biomarker to clinical use has been investigated, however, existing literature has primarily focused on PDE4D7 mRNA expression in patient samples or protein expression in prostate cancer cell lines, neglecting PDE4D7 protein expression in patient samples essential for clinical translation.

The present invention aims to overcome these issues, among other by the methods and uses as defined in the appended claims.

SUMMARY OF THE INVENTION

In a first aspect the invention relates to a computer implemented method for predicting an outcome for a patient with hormone sensitive prostate cancer, the method comprising: receiving a quantification of PDE4D7 protein in the nuclei of cells in a sample obtained from the patient; wherein the quantification is low or high nuclear presence of PDE4D7, and wherein low nuclear presence of PDE4D7 indicates a poor expected outcome and high nuclear presence of PDE4D7 status indicates a good expected outcome.

In a second aspect the invention relates to a method for predicting an outcome for a patient with hormone sensitive prostate cancer, the method comprising: performing a quantification of PDE4D7 protein in the nuclei of cells in a sample obtained from the patient; wherein the quantification is low or high nuclear presence of PDE4D7, and wherein low nuclear presence of PDE4D7 indicates a poor expected outcome and high nuclear presence of PDE4D7 status indicates a good expected outcome.

In a third aspect the invention relates to a computer program product comprising instructions which when the program is executed by a computer cause the computer to carry out a method for predicting an outcome for a patient with hormone sensitive prostate cancer comprising the steps as defined in the first or the second aspect of the invention.

In a fourth aspect the invention relates to an apparatus for predicting an outcome for a patient with hormone sensitive prostate cancer, comprising: an input adapted to receive a quantification of PDE4D7 protein in the nuclei of cells in a sample obtained from the patient; a processor adapted to determine the prediction of outcome based on the quantification of PDE4D7 protein in the nuclei of cells in a sample, and optionally, a providing unit adapted to provide the prediction to a medical caregiver or the subject.

In a fifth aspect the invention relates to a therapy for use in the treatment, alleviation or prevention of hormone sensitive prostate cancer in a patient, the use comprising predicting an outcome for the patient based on a quantification of PDE4D7 protein in the nuclei of cells in a sample obtained from the patient, and administering the therapy to the patient based on the outcome, wherein when the outcome is favorable the therapy is a monotherapy selected from surgery, radiation therapy, hormonal treatment or chemotherapy, and wherein when the outcome is not favorable the therapy is a combination therapy comprising at least one therapy selected from surgery, radiation therapy, hormonal treatment, chemotherapy, immunotherapy or radiopharmaceutical based therapy; wherein the patient is a patient with hormone sensitive prostate cancer.

In a sixth aspect the invention relates to a kit of parts comprising a PDE4D7 specific antibody.

In a seventh aspect the invention relates to use of the kit of parts according to the sixth aspect of the invention in quantifying PDE4D7 protein in the nuclei of cells in a sample, the method comprising: incubating the sample with the PDE4D7 specific antibody;

binding the stained sample with a suitable secondary antibody; detecting PDE4D7 by visualizing the secondary antibody; and and detecting and optionally quantifying the nuclear localization of PDE4D7.

BRIEF DESCRIPTION OF THE DRAWINGS

Embodiments of the invention are further described hereinafter with reference to the accompanying drawings, in which:

Fig 1. depicts a scatter plot showing correlation between independent observers' weighted histoscores in weighted histoscore units (WHU) for nuclear PDE4D7 expression in hormone-sensitive and castrate-resistant prostate cancer cohort.

Fig. 2 depicts a Bland-Altman plot showing mean and difference in weighted histoscores measured in weighted histoscore units (WHU) for nuclear PDE4D7 expression in hormone-sensitive and castrate-resistant prostate cancer cohort.

Fig. 3 depicts representative images of (A) weak, (B) moderate, and (C) strong nuclear, cytoplasmic and membranous PDE4D7 staining in hormone-sensitive and castrate-resistant prostate cancer cohort. 10X magnification.

Fig. 4 depicts a histogram showing hormone-sensitive nuclear PDE4D7 expression weighted histoscores in weighted histoscore units (WHU). The curve shows the normal distribution.

Fig. 5 depicts a Kaplan-Meier survival analysis showing association between nuclear PDE4D7 expression and time to biochemical relapse in hormone-sensitive tumours. Nuclear PDE4D7 expression: Low (n=8) mean TTR=2.505 years (95% CI 1.298-3.712). High (n=8) mean TTR=4.034 years (95% CI 2.417-5.651).

Fig. 6 depicts a Kaplan-Meier survival analysis showing association between nuclear PDE4D7 expression and cancer-specific survival in hormone-sensitive tumours. Nuclear PDE4D7 expression: Low (n=8) mean CSS=5.828 years (95% CI 4.050-7.606). High (n=8) mean CSS=10.045 years (95% CI 8.533-11.557).

Fig. 7 depicts a histogram showing castrate-resistant nuclear PDE4D7 expression weighted histoscores in weighted histoscore units (WHU). The curve shows the normal distribution.

Fig. 8 depicts a Kaplan-Meier survival analysis showing association between nuclear PDE4D7 expression and time to death from biochemical relapse in castrate-resistant tumours. Nuclear PDE4D7 expression: Low (n=9) mean TTDR=3.851 years (95% CI 2.449-5.253). High (n=10) mean TTDR=4.183 years (95% CI 2.840-5.527).

Fig. 9 depicts a Kaplan-Meier survival analysis showing association between nuclear PDE4D7 expression and cancer-specific survival in castrate-resistant tumours. Nuclear PDE4D7 expression: Low (n=9) mean CSS=7.403 years (95% CI 5.614-9.193). High (n=10) mean CSS=7.040 years (95% CI 5.005-9.074).

Fig. 10 depicts a scatter plot showing correlation between independent observers' weighted histoscores in weighted histoscore units (WHU) for cytoplasmic PDE4D7 expression in hormone-sensitive and castrate-resistant prostate cancer cohort.

Fig. 11 depicts a Bland-Altman plot showing mean and difference in weighted histoscores measured in weighted histoscore units (WHU) for cytoplasmic PDE4D7 expression in hormone-sensitive and castrate-resistant prostate cancer cohort.

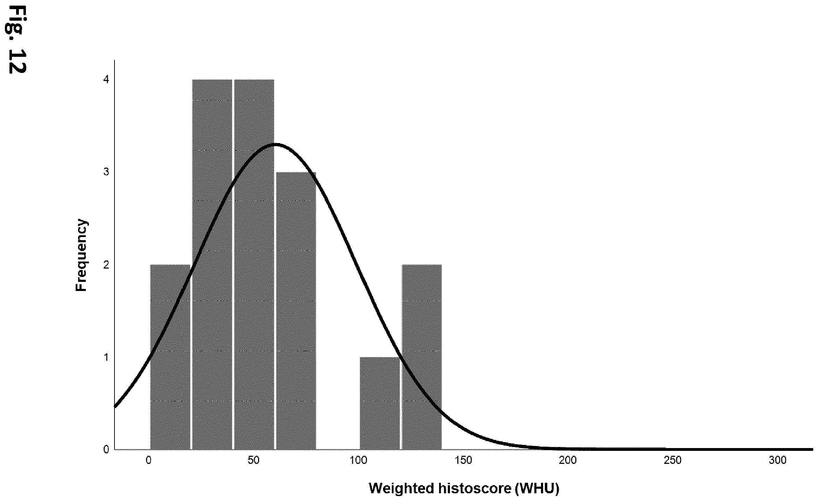

Fig. 12 depicts a histogram showing hormone-sensitive cytoplasmic PDE4D7 expression weighted histoscores in weighted histoscore units (WHU). The curve shows the normal distribution.

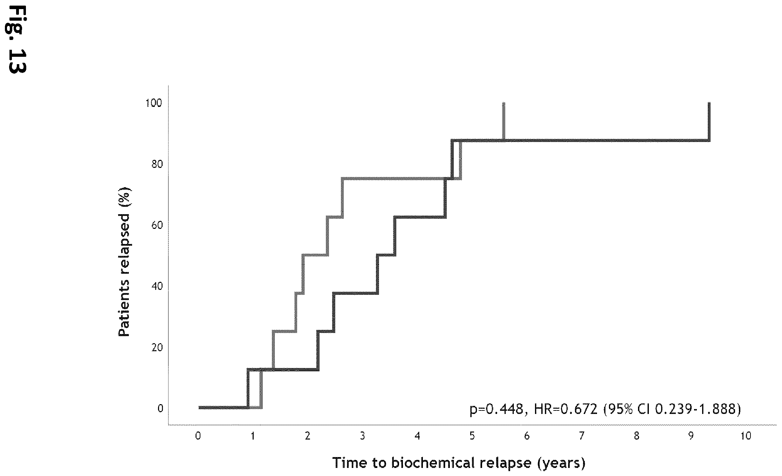

Fig. 13 depicts a Kaplan-Meier survival analysis showing association between cytoplasmic PDE4D7 expression and time to biochemical relapse in hormone-sensitive tumours. Cytoplasmic PDE4D7 expression: Low (n=8) mean TTR=2.686 years (95% CI 1.562-3.811). High (n=8) mean TTR=3.852 years (95% CI 2.099-5.606).

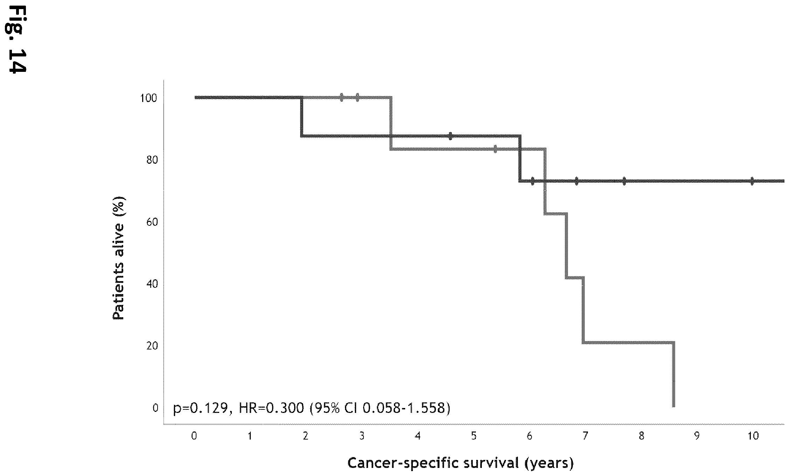

Fig. 14 depicts a Kaplan-Meier survival analysis showing association between cytoplasmic PDE4D7 expression and cancer-specific survival in hormone-sensitive tumours. Cytoplasmic PDE4D7 expression: Low (n=8) mean CSS=6.508 years (95% CI 5.065-7.951). High (n=8) mean CSS=9.028 years (95% CI 6.742-11.314).

Fig. 15 depicts a Histogram showing the range of weighted histoscores in weighted histoscore units (WHU) for cytoplasmic PDE4D7 expression in castrate-resistant tumours. The curve shows the normal distribution.

Fig. 16 depicts a Kaplan-Meier survival analysis showing association between cytoplasmic PDE4D7 expression and time to death from biochemical relapse in castrate-resistant tumours. Cytoplasmic PDE4D7 expression: Low (n=9) mean TTDR=3.439 years (95% CI 2.426-4.451). High (n=10) mean TTDR=4.421 years (95% CI 2.729-6.113).

Fig. 17 depicts a Kaplan-Meier survival analysis showing association between cytoplasmic PDE4D7 expression and cancer-specific survival in castrate-resistant tumours. Cytoplasmic PDE4D7 expression: Low (n=9) mean CSS=7.074 years (95% CI 5.535-8.614). High (n=10) mean CSS=7.406 years (95% CI 5.071-9.741).

Fig. 18 depicts a scatter plot showing correlation between independent observers' weighted histoscores in weighted histoscore units (WHU) for membranous PDE4D7 expression in hormone-sensitive and castrate-resistant prostate cancer cohort.

Fig. 19 depicts a Bland-Altman plot showing mean and difference in weighted histoscores measured in weighted histoscore units (WHU) for membranous PDE4D7 expression in hormone-sensitive and castrate-resistant prostate cancer cohort.

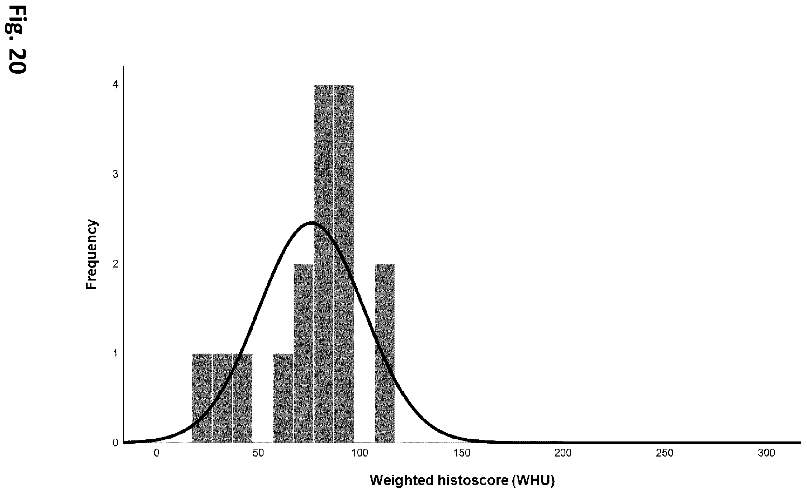

Fig. 20 depicts a histogram showing hormone-sensitive membranous PDE4D7 expression weighted histoscores in weighted histoscore units (WHU). The curve shows the normal distribution.

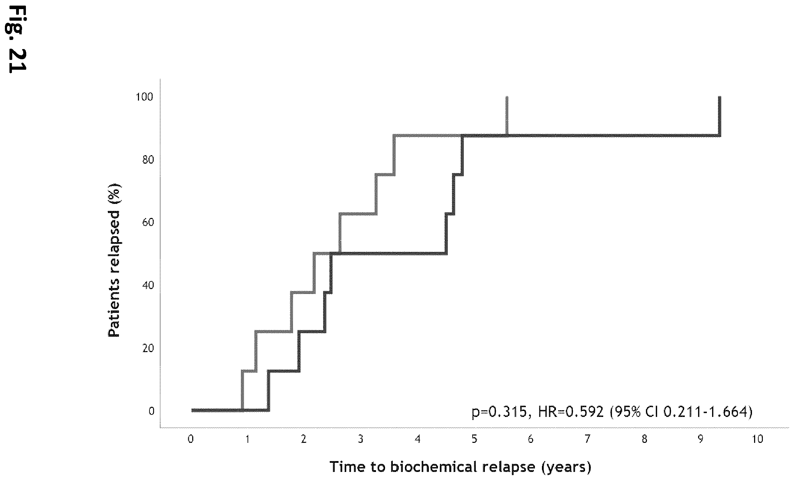

Fig. 21 depicts a Kaplan-Meier survival analysis showing association between membranous PDE4D7 expression and time to biochemical relapse in hormone-sensitive tumours. Membranous PDE4D7 expression: Low (n=8) mean TTR=2.626 years (95% CI 1.575-3.677). High (n=8) mean TTR=3.913 years (95% CI 2.137-5.688).

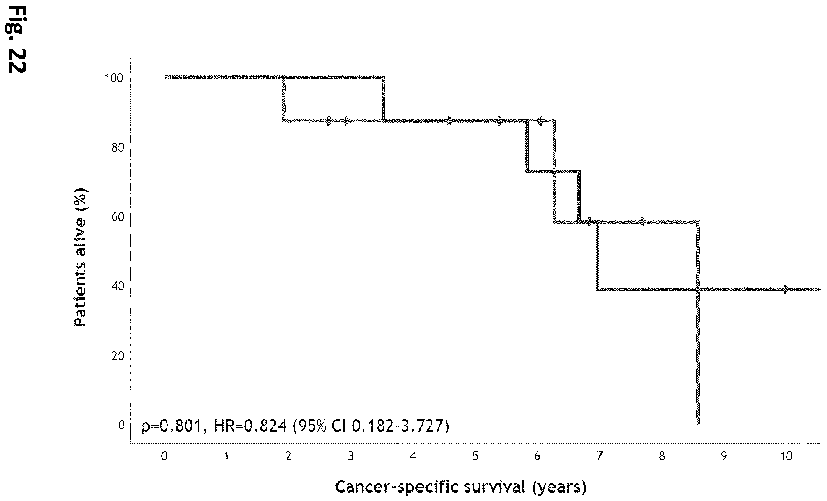

Fig. 22 depicts a Kaplan-Meier survival analysis showing association between membranous PDE4D7 expression and cancer-specific survival in hormone-sensitive tumours. Membranous PDE4D7 expression: Low (n=8) mean CSS=7.064 years (95% CI 4.948-9.180). High (n=8) mean CSS=7.844 years (95% CI 5.835-9.853).

Fig. 23 depicts a histogram showing castrate-resistant membranous PDE4D7 expression weighted histoscores in weighted histoscore units (WHU). The curve shows the normal distribution.

Fig. 24 depicts a Kaplan-Meier survival analysis showing association between membranous PDE4D7 expression and time to death from biochemical relapse in castrate-resistant tumours. Membranous PDE4D7 expression: Low (n=10) mean TTDR=3.831 years (95% CI 2.965-4.698). High (n=9) mean TTDR=3.750 years (95% CI 1.706-5.794).

Fig. 25 depicts a Kaplan-Meier survival analysis showing association between membranous PDE4D7 expression and cancer-specific survival in castrate-resistant tumours. Membranous PDE4D7 expression: Low (n=10) mean CSS=7.224 years (95% CI 5.821-8.628). High (n=9) mean CSS=7.200 years (95% CI 4.751-9.649).

DEFINITIONS

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular forms "a," "an" and "the" include plural referents unless the context clearly dictates otherwise. For example, a method for administrating a pharmaceutical agent includes the administrating of a plurality of molecules (e.g., 10's, 100's, 1000's, 10's of thousands, 100's of thousands, millions, or more molecules).

As used herein, "about" and "approximately", when referring to a measurable value such as an amount, a temporal duration, and the like, is meant to encompass variations of $\pm 20\%$ or $\pm 10\%$, more preferably $\pm 5\%$, even more preferably $\pm 1\%$, and still more preferably $\pm 0.1\%$ from the specified value, as such variations are appropriate to perform the disclosed invention. Unless otherwise clear from context, all numerical values provided herein include numerical values modified by the term "about."

As used herein, "and/or" refers to a situation wherein one or more of the stated cases may occur, alone or in combination with at least one of the stated cases, up to with all of the stated cases.

As used herein, "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, "comprising" or "to comprise" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of".

As used herein, "conventional techniques" or "methods known to the skilled person" refer to a situation wherein the methods of carrying out the conventional techniques used in methods of the invention will be evident to the skilled worker. The practice of conventional techniques in molecular biology, biochemistry, cell culture, genomics, sequencing, medical treatment, pharmacology, immunology and related fields are well-known to those of skill in the art and are discussed, in various handbooks and literature references.

As used herein, "exemplary" or "for example" means "serving as an example, instance, or illustration," and should not be construed as excluding other configurations, including those disclosed herein.

Throughout this disclosure, various aspects of the invention can be presented in a range format. It should be understood that the description in range format is merely for convenience and should not be construed as a limitation on the scope of the invention. The description of a range should be considered to have specifically disclosed all the possible subranges as well as individual numerical values within that range including both integers and non-integers. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, 6 etc. This applies regardless of the breadth of the range.

As used herein, "cancer" refers to the physiological condition in mammals that is typically characterized by unregulated cell growth. The terms "cancer," "neoplasm," and "tumor," are often used interchangeably to describe cells that have undergone a malignant transformation that makes them pathological to the host organism. Primary cancer cells can be distinguished from non-cancerous cells by techniques known to the skilled person. A cancer cell, as used herein, includes not only primary cancer cells, but also cancer cells derived from such primary cancer cell, including metastasized (secondary) cancer cells, and cell lines derived from cancer cells. Examples include solid tumors and non-solid tumors or blood tumors. Treatment of a cancer in a subject includes the treatment of a tumor in the subject.

The term "prostate cancer" refers to a cancer of the prostate tissue, which occurs when cells in the prostate mutate and begin to grow out of control.

The term "prostate cancer specific death or disease specific death" refers to death of a patient from a prostate cancer.

The term "clinical recurrence" refers to the presence of clinical signs indicating the presence of tumour cells as measured, for example using in vivo imaging.

The term "metastases" refers to the presence of metastatic disease in organs other than a prostate tissue.

As used herein, a "subject" is to indicate the organism to be treated e.g., to which administration is contemplated. The subject may be any subject in accordance with the present invention, including, but not limited to humans, males, females, infants, children, adolescents, adults, young adults, middle-aged adults or senior adults and/or other primates or mammals. Preferably the subject is a human patient. In some embodiments, the subject may have been diagnosed with cancer, an immune related disorder, a bleeding disorder, a disorder related to over expression of a protein, a disorder related to under expression of a protein. In some embodiments the subject may be at risk of developing a disease or disorder which may be prevented or ameliorated by vaccination.

As used herein, "treatment", "treating", "palliating", "alleviating" and "ameliorating" in the context of a subject to be treated, all refer to an approach for obtaining beneficial or desired results including, but not limited to, therapeutic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient can still be afflicted with the underlying disorder. As used herein, "prevention" and "preventing" refers to an approach for reducing in part or in full the change of developing adverse effects, for example normally associated with the use of a particular drug or agent. Within the context of the current invention, for example, the terms may refer to preventing, treating or reducing the effects of cancer, an immune related disorder, a bleeding disorder, a disorder related to over expression of a protein, a disorder related to under expression of a protein. In some embodiments the preventing may also refer to the effects of preventing a disease by vaccination.

As used herein the term "nucleic acid" or "polynucleotide" refers to any polymers or oligomers of (contiguous) nucleotides. The nucleic acid may be DNA or RNA, or a mixture thereof, and may exist permanently or transitionally in single-stranded or double-stranded form, including homoduplex, heteroduplex, and hybrid states. The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogeneous or homogenous in composition, and may be isolated from naturally occurring sources or may be artificially or synthetically produced. The term "isolated" thus means isolated from naturally occurring sources or artificially or synthetically produced.

As used herein, "Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, Calif., or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

As used herein, the terms "protein" and "polypeptide" refer to molecules consisting of a chain of amino acids, without reference to a specific mode of action, size, three dimensional structure or origin. A "fragment" or "portion" or "part" of a polypeptide may thus still be referred to as a "polypeptide". An "isolated protein" or isolated polypeptide" is used to refer to a protein or polypeptide which is no longer in its natural environment, for example in vitro or in a recombinant host cell.

As used herein the term "construct" or "nucleic acid construct" or "vector" refers to a man-made nucleic acid molecule resulting from the use of recombinant DNA technology and which is used to deliver exogenous DNA into a host cell, often with the purpose of expression in the host cell of a DNA region comprised on the construct. The vector backbone of a construct may for example be a plasmid into which a (chimeric) gene is integrated or, if a suitable transcription regulatory sequence is already present, only a desired nucleic acid sequence (e.g. a coding sequence) is integrated downstream of the transcription regulatory sequence. Vectors may comprise further genetic elements to facilitate their use in molecular cloning, such as e.g. selectable markers, multiple cloning sites and the like.

DETAILED DESCRIPTION OF THE INVENTION

The invention is defined herein, and in particular in the accompanying claims. Subject-matter which is not encompassed by the scope of the claims does not form part of the present claimed invention.

It is contemplated that any method, use or composition described herein can be implemented with respect to any other method, use or composition described herein. Embodiments or preferences discussed in the context of methods, use and/or compositions of the invention may likewise be employed with respect to any other method, use or composition described herein. Thus, an embodiment or preference pertaining to one method, use or composition may be applied to other methods, uses and compositions of the invention as well.

Any references in the description to methods of treatment refer to the compounds, pharmaceutical compositions and medicaments of the present invention for use in a method for treatment of the human (or animal) body by therapy.

As embodied and broadly described herein, the present invention is directed to the surprising finding that low or high nuclear localization of PDE4D7 is predictive for an outcome for patients with hormone sensitive prostate cancer. The inventors used immunohistochemistry staining with a PDE4D7 specific antibody in samples obtained from hormone sensitive prostate cancer patients and castration resistant prostate cancer patients. Using this imaging data subcellular localization of PDE4D7 was analyzed. In particular, the inventors analyzed nuclear, membranous and cytosolic localization within the two patient groups and surprisingly found that high nuclear localization of PDE4D7 is indicative of long survival time in hormone sensitive prostate cancer while low nuclear localization of PDE4D7 is indicative of a poor outcome (short cancer specific survival time). Interestingly nuclear localization of PDE4D7 is not predictive in castrate resistant prostate cancer, nor is localization of membranous or cytosolic PDE4D7 in either hormone sensitive or castrate resistant prostate cancer.

Expression levels of PDE4D7 have been described as prognostic in prostate cancer, among others by the inventors and others (Böttcher et al., Br J Cancer. 2015 Nov 17;113(10):1502-11). The present finding may provide a further improved prognosis for hormone resistant prostate cancer patients or may provide an alternative prognostic tool in case no reliable sequencing data is available.

The present invention relies on determining the subcellular localization, in particular nuclear localization of PDE4D7 and correlating the nuclear localization of PDE4D7 to an outcome for hormone sensitive prostate cancer patients. In particular, high nuclear localization of PDE4D7 is indicative of a good outcome while low nuclear localization of PDE4D7 is indicative of a poor outcome. For these reasons, the inventors anticipate that any method that allows for the quantification of nuclear PDE4D7 protein may be used to predict the outcome as described herein.

The invention as broadly described herein provides an alternative method for predicting an outcome in hormone sensitive prostate cancer patients. This may be advantageous for several reasons: the present methods may complement described methods (e.g. determining PDE4D7 expression levels) to provide a more accurate prediction; otherwise the present method provides an alternative method which may provide superior prediction or may be used if reliable sequencing data is absent (so no accurate expression levels can be determined).

Therefore, in a first aspect the invention relates to a computer implemented method for predicting an outcome for a patient with hormone sensitive prostate cancer, the method comprising: receiving a quantification of PDE4D7 protein in the nuclei of cells in a sample obtained from the patient; wherein the quantification is low or high nuclear presence of PDE4D7, and wherein low nuclear presence of PDE4D7 indicates a poor expected outcome and high nuclear presence of PDE4D7 status indicates a good expected outcome.

In a second aspect the invention relates to a method for predicting an outcome for a patient with hormone sensitive prostate cancer, the method comprising: performing a quantification of PDE4D7 protein in the nuclei of cells in a sample obtained from the patient; wherein the quantification is low or high nuclear presence of PDE4D7, and wherein low nuclear presence of PDE4D7 indicates a poor expected outcome and high nuclear presence of PDE4D7 status indicates a good expected outcome.

It is understood that the prediction of an outcome for a patient with hormone sensitive prostate cancer is based on nuclear localization of PDE4D7 protein, and therefore quantification of (nuclear PDE4D7 in a prostate cancer sample of a patient with hormone sensitive prostate cancer may be used to predict the outcome.

PDE4D7 is a splice variant of the PDE4D gene, which was identified by the inventors as an important biomarker in prostate cancer. Loss of PDE4D7 expression in prostate cancer was demonstrated with a poor disease outcome, such as androgen independent growth and therapy resistance (Gulliver et al. Loss of PDE4D7 expression promotes androgen independence, neuroendocrine differentiation and alterations in DNA repair: implications for therapeutic strategies. Br J Cancer. 2023 Oct;129(9):1462-1476.). The term "phosphodiesterase 4D7" or "PDE4D7" refers to the splice variant 7 of the human phosphodiesterase PDE4D, i.e., the human phosphodiesterase PDE4D7 gene, for example, to the sequence as defined in NCBI Reference Sequence: NM_001165899.1, specifically, to the nucleotide sequence as set forth in SEQ ID NO:1, which corresponds to the sequence of the above indicated NCBI Reference Sequence of the PDE4D7 transcript, and also relates to the corresponding amino acid sequence for example as set forth in SEQ ID NO:2, which corresponds to the protein sequence defined in NCBI Protein Accession Reference Sequence NP_001159371.1 encoding the PDE4D7 polypeptide.

The term "quantification of PDE4D7 protein in the nuclei of cells" as used herein refers to the abundance of PDE4D7 protein in the nucleus of a prostate cancer sample obtained from a subject. PDE4D7 may abundant (have high nuclear presence) or not abundant (have low nuclear presence) in the nuclei of a sample form the subject. It is understood that the relative terms may be with reference to a predetermined threshold. The skilled person appreciates that such threshold can easily be determined in reference samples obtained from prostate cancer samples, e.g. hormone sensitive prostate cancer samples obtained from patients. It is understood that the threshold will be preferably determined using the same methodology.

For example, when PDE4D7 protein in the nuclei of cells in a sample obtained from the patient is quantified using a weighted histoscore on samples stained with immunohistochemistry and a PDE4D7 specific antibody, the threshold should be preferably predetermined in reference samples using the same methods. The threshold may be set for example as the mean value obtained, the average value obtained or can be set by specifically correlating the outcomes to the determined individual values to determine an optimal threshold able to separate the different outcomes. It is understood that other methods for quantification of PDE4D7 protein in the nuclei of cells in a sample obtained from the patient and for setting a predetermined threshold are known so the invention should not be construed as limited to the provided example.

As the prediction of an outcome for a patient with hormone sensitive prostate cancer is based on the quantification of PDE4D7 protein in the nuclei of cells in a sample obtained from the patient, any method allowing quantification of PDE4D7 protein in the nuclei of cells in the sample may be used in the methods described herein. Non limiting examples comprise: antibody based methods, or mass spectrometry based methods, as described below.

Antibody based methods may be used for example on tissue sections of prostate cancer samples. For example a PDE4D7 specific antibody can be used as a primary antibody followed by staining with a suitable secondary antibody to perform immunohistochemistry to allow determination and quantification of PDE4D7 protein in the nuclei of the sample. Such methods are known to the skilled person. For example the secondary antibody can be conjugated to enzymes such as horseradish peroxidase (HRP) or alkaline phosphatase (AP); or fluorescent dyes such as fluorescein isothiocyanate (FITC), rhodamine derivatives, Alexa Fluor dyes; or other molecules to be used in various applications such as but not limited to ELISA, Western blot, Immunostaining, Immunohistochemistry and Immunocytochemistry. In a further example, cell nuclei can be isolated from a cell sample and PDE4D7 protein can be quantified in the isolated nuclei using techniques such as ELISA or Western blot.

Thus the methods as broadly described herein may be based on a quantification, or the quantification is part of the method, wherein the quantification comprises determining the amount of nuclear PDE4D7 protein. In particular the amount is determined with reference to a predetermined threshold and the quantification includes assigning a low or high nuclear presence qualitative value to the quantification. The quantification may for example assign absolute values as may be done using ELISE assays where protein content can be measured and quantified when using a reliable protein standard. The quantification may also be performed in relative or arbitrary units, for example using immunofluorescence techniques where quantification can be based on light emitted or pixel intensity of an image.

Therefore, in an embodiment the quantification of PDE4D7 protein in the nuclei of cells is performed using an antibody based technology or using a mass spectrometry based technology. Preferably the quantification of PDE4D7 protein in the nuclei of cells is performed by immunohistochemistry staining or immunofluorescence staining of the sample, ELISA, Western blot or mass spectrometry analysis on isolated nuclei from the sample, or by performing mass spectrometry imaging on the sample.

In an embodiment the quantification may be based on determining a weighted histoscore. The skilled person is aware how a weighted histoscore can be determined, as for example described in Rizzardi et al., Diagn Pathol 7, 42 (2012), incorporated herewith by reference in its entirety. For example the weighted histoscore may be based on image analysis of immunohistochemistry staining of a sample. A weighted histoscore may be assigned manually be an observer or it may be automated using an image analysis algorithm. For example, a histoscore may be calculated by assigning different values to different staining intensities. For the purpose of the present invention the staining intensity would be nuclear localized PDE4D7. Nuclear localization may be based on morphology or by co-staining with a nuclear marker. For example the staining intensities may be divided in no staining, mild staining or strong staining, or alternatively in no staining, mild staining, medium staining and strong staining. The weighted histoscore may then be determined by assigning a value to each score and determining the percentage of cells for each score in the sample. For example the score could be calculated using the formula:

$$0\times(\% \text{ cells not stained})+1\times(\% \text{ cells weakly stained in the nucleus})+2\times(\% \text{ cells}$$

$$\text{moderately stained in the nucleus})+3\times(\% \text{ cells strongly stained in the nucleus})$$

In such case the theoretical highest value is 300 (100% of cells have strong staining) and theoretical lowest value is 0 where all cells have no staining. It is however understood that values assigned to each condition may be varied and are not fixed to 0, 1, 2, and 3. As mention, the weighted histoscore can be determined manually be an observer assigning the scores to each cell in a sample (e.g. a immunohistochemistry staining of a prostate cancer tissue section). For accuracy it is then preferable that part or all of the samples or cells are scored by a second observer to increase accuracy and reduce bias. Alternatively a computer algorithm may be used to perform mage analysis and assign a weighted histoscore. An exemplary program is QuPath, which is a software used for digital pathology analysis featuring algorithms for cell detection and interactive machine learning for object classification. For example the object classifier (Random trees) may be trained by a subset of around three images. A small circular annotation containing both tumour and stroma may be created on each image. Preferably any areas of folded tissue or out of focus tissue are removed from the ROI. A preprocessing step may be performed to increase the quality of the staining using automatic estimated stain vectors. Watershed cell detection may be performed followed by training the object classifier by annotating a small region of tumour and of stroma. Intensity thresholds may be set by manually identifying a cell classified as weakly, moderately and strongly stained. The training classifier may be saved and used for all images for the same protein to ensure consistency. A number of ROIs, for example five, may be annotated on each of the images in the full set and the training classifier was applied. The "tumor H-score" (Histoscore) can be exported for each image.

Thus in an embodiment the quantification is determined by calculating a weighted histoscore for nuclear localization of PDE4D7 in the nuclei of the sample. In an embodiment the weighted histoscore is calculated using the formula:

$$0\times(\% \text{ cells not stained})+1\times(\% \text{ cells weakly stained in the nucleus})+2\times(\% \text{ cells}$$

$$\text{moderately stained in the nucleus})+3\times(\% \text{ cells strongly stained in the nucleus}).$$

In an embodiment low or high nuclear presence of PDE4D7 protein in the nuclei of cells in the sample is determined when the weighted histoscore is below or above the mean reference value, wherein the mean reference value is predetermined in a cohort of reference samples, preferably prostate cancer reference samples, more preferably hormone sensitive prostate cancer reference samples. In an embodiment low or high nuclear presence of PDE4D7 protein in the nuclei of cells in the sample is determined when the weighted histoscore is below or above a preset reference value, preferably wherein the reference value is predetermined in a cohort of reference samples, preferably prostate cancer reference samples, more preferably hormone sensitive prostate cancer reference samples, preferably wherein the preset value is a weighted histoscore of 90.

Prostate cancer is the uncontrolled growth of cells in the prostate, a gland in the male reproductive system below the bladder. Early prostate cancer causes no symptoms. Most cases are detected after screening tests - typically blood tests for levels of prostate-specific antigen (PSA) - indicate unusual growth of prostate tissue. Diagnosis requires a biopsy of the prostate. If cancer is present, standard of practice is assigning a Gleason score, with a higher score representing a more dangerous tumor. Medical imaging may be performed to identify metastases. Based on the Gleason score, PSA levels, and imaging results, a cancer case is assigned a stage 1 to 4. Higher stage signifies a more advanced, more dangerous disease. In hormone sensitive prostate cancer tumor cells require androgens for growth, meaning tumor growth can be controlled by lowering androgen levels. Hormone sensitive prostate cancer may be metastatic or non-metastatic. Examples of therapies for lowering androgen levels are Analogs of gonadotropin-releasing hormone (GnRH), antiandrogens, progestogens and estrogens.

In one embodiment, the diagnosing, monitoring, prognosticating, stratifying risk, and providing a recommendation as mentioned herein is to be carried out on a biological sample obtained from an individual or subject. The term "biological sample" or "sample obtained from an subject" refers to any biological material obtained via suitable methods known to the person skilled in the art from an individual. The biological sample used may be collected in a clinically acceptable manner, e.g., in a way that proteins and their subcellular localization are preserved.

The biological sample(s) may include body tissue and/or a fluid, such as, but not limited to, blood, sweat, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male individual. Additionally, cells may be purified from obtained body tissues and fluids if necessary, and then used as the biological sample. In some embodiments, the sample is a tissue sample, a urine sample, a urine sediment sample, a blood sample, a saliva sample, a semen sample, a sample including circulating tumor cells, extracellular vesicles, a sample containing prostate secreted exosomes, or cell lines or cancer cell line. Preferably the samples are prostate cancer samples or samples obtained from a metastasis of a prostate tumor. Such samples may for example be collected by a biopsy. Thus in an embodiment the sample is a prostate cancer sample or a metastasis of a prostate tumor. In an embodiment the sample is a biopsy.

In one embodiment, biopsy or resections samples may be obtained and/or used. Such samples may include cells or cell lysates.

In a specific embodiment, the content of a biological sample may also be submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below. In a particularly advantageous embodiment the sample is treated to isolate the nuclei of the cells in the sample.

The method described herein is particularly useful for predicting an outcome for a subject having hormone sensitive prostate cancer. In an embodiment the hormone sensitive prostate cancer is metastatic hormone sensitive prostate cancer. In an embodiment the outcome is cancer specific survival. In an alternative embodiment the outcome is cancer free survival time, 5 year survival rate, time until recurrence or time until biochemical relapse.

In an embodiment the method is used to recommend a treatment o treatment strategy for the subject. In an embodiment the treatment recommendation is provided to a medical caregiver.

In an embodiment the method is combined with determining the expression levels of a biomarker which is useful in providing a prediction for an outcome in prostate cancer. In an embodiment the biomarker is useful in predicting an outcome in hormone sensitive prostate cancer. In an embodiment the biomarker is PDE4D, PDE4D5, or PDE4D7 and/or a combination thereof.

In an embodiment the methods as broadly described herein are further combined with a clinical parameter. In an embodiment the clinical parameter is a Gleason score and/or a National Comprehensive Cancer Network (NCCN) classification, such as one or more of very low risk (VLR), low risk (LR), favorable intermediate risk (FIR), unfavorable intermediate risk (UIR), and high risk (HR). The combined prognostic risk score may be determined with a regression function derived from subjects that have been monitored for prostate cancer.

In a third aspect the invention relates to a computer program product comprising instructions which when the program is executed by a computer cause the computer to carry out a method for predicting an outcome for a patient with hormone sensitive prostate cancer comprising the steps as defined in the first or the second aspect of the invention. As envisioned herein the computer program product may be inputted with a quantification of PDE4D7 protein in the nuclei of cells in a sample obtained from the patient as broadly described herein.

In a fourth aspect the invention relates to an apparatus for predicting an outcome for a patient with hormone sensitive prostate cancer, comprising: an input adapted to receive a quantification of PDE4D7 protein in the nuclei of cells in a sample obtained from the patient; a processor adapted to determine the prediction of outcome based on the quantification of PDE4D7 protein in the nuclei of cells in a sample, and optionally, a providing unit adapted to provide the prediction to a medical caregiver or the subject. As envisioned herein the apparatus may be inputted with a quantification of PDE4D7 protein in the nuclei of cells in a sample obtained from the patient as broadly described herein.

As described herein high nuclear presence of PDE4D7 protein indicates a favourable outcome for the subject while low nuclear presence of PDE4D7 protein (in cells of a prostate cancer sample or a metastasis thereof) indicates a non-favourable outcome. For example high nuclear presence of PDE4D7 indicates a greater likelihood of survival while low nuclear presence of PDE4D7 protein indicates increased chance of cancer specific death. Therefore, nuclear presence of PDE4D7 protein in a sample from a subject having hormone sensitive prostate cancer, or its associated outcome, may be used in selecting a therapy for the subject. In general high nuclear presence of PDE4D7 corresponds to a favourable outcome, and therefore warrants a milder therapy as opposed to low nuclear presence of PDE4D7.

Thus in a fifth aspect the invention relates to a therapy for use in the treatment, alleviation or prevention of hormone sensitive prostate cancer in a patient, the use comprising predicting an outcome for the patient based on a quantification of PDE4D7 protein in the nuclei of cells in a sample obtained from the patient, and administering the therapy to the patient based on the outcome, wherein when the outcome is favorable the therapy is a monotherapy selected from surgery, radiation therapy, hormonal treatment or chemotherapy, and wherein when the outcome is not favorable the therapy is a combination therapy comprising at least one therapy selected from surgery, radiation therapy, hormonal treatment, chemotherapy, immunotherapy or radiopharmaceutical based therapy; wherein the patient is a patient with hormone sensitive prostate cancer. Alternatively the invention relates to a method of treatment comprising predicting an outcome for the patient based on a quantification of PDE4D7 protein in the nuclei of cells in a sample obtained from the patient, and administering a therapy to the patient based on the outcome. Preferably when the outcome is favorable the therapy is a monotherapy selected from surgery, radiation therapy, hormonal treatment or chemotherapy, and when the outcome is not favorable the therapy is a combination therapy comprising at least one therapy selected from surgery, radiation therapy, hormonal treatment, chemotherapy, immunotherapy or radiopharmaceutical based therapy.

Thus in an embodiment when high nuclear presence of PDE4D7 is observed (indicating a favorable outcome) in the patient sample (of a patient with hormone sensitive prostate cancer), the administered or recommended treatment is a monotherapy selected from surgery, radiation therapy, hormonal treatment or chemotherapy. In an embodiment when low nuclear presence of PDE4D7 is observed (indicating a non favorable outcome) in the patient sample (of a patient with hormone sensitive prostate cancer), the administered or recommended treatment is a combination therapy comprising at least one therapy selected from surgery, radiation therapy, hormonal treatment, chemotherapy, immunotherapy or radiopharmaceutical based therapy.

In an embodiment the outcome is determined by the methods, apparatus, computer program product, kit or uses as broadly described herein.

In an embodiment when high nuclear presence of PDE4D7 is found or a favourable outcome is predicted, the administered or recommended treatment is a monotherapy of a standard of care treatment for hormone sensitive prostate cancer. In an embodiment the treatment is administered in a low dose or a short duration. For example when administering anti-androgens the duration of treatment may be 6-12 weeks (short treatment). For example when providing radiation therapy a boost can be omitted to reduce the dose of the treatment. The monotherapy may be selected from surgery, radiation therapy, hormonal treatment or chemotherapy.

In an embodiment when low nuclear presence of PDE4D7 is found or a non-favourable outcome is predicted, the administered or recommended treatment is a combination therapy of a standard of care treatment for hormone sensitive prostate cancer. In an embodiment the treatment is administered in a high dose or a long duration. For example when administering anti-androgens the duration of treatment may be 2 years (long treatment). For example when providing radiation therapy a boost can be provided to increase the dose of the treatment. The combination therapy comprising at least one therapy selected from surgery, radiation therapy, hormonal treatment, chemotherapy, immunotherapy or radiopharmaceutical based therapy. In particularly preferred embodiments the combination therapy is selected from the following combinations: surgery and radiation therapy; surgery and hormone therapy (with or without radiation therapy); radiation therapy and hormone therapy; hormone therapy and chemotherapy; hormone therapy and immunotherapy; or hormone therapy and radiopharmaceutical based therapy.

Non limiting examples of treatment by surgery comprise radical prostatectomy, open prostatectomy, Radical retropubic prostatectomy, Radical perineal prostatectomy, Laparoscopic prostatectomy, Laparoscopic radical prostatectomy, and Robotic prostatectomy.

Non limiting examples of radiation therapy include External beam radiation therapy (EBRT), Three-dimensional conformal radiation therapy (3D-CRT), Intensity modulated radiation therapy (IMRT), Stereotactic body radiation therapy (SBRT),

MRI-guided radiation therapy, Proton beam radiation therapy, Brachytherapy (internal radiation therapy), Permanent (low dose rate, or LDR) brachytherapy, or Temporary (high-dose rate, or HDR) brachytherapy.

Non limiting examples of hormonal treatment therapy (also interchangeable referred herein as hormone therapy or anti-androgen therapy) are LHRH agonists (such as but not limited to Leuprolide (Lupron, Eligard), leuprolide mesylate (Camcevi), Goserelin (Zoladex), Triptorelin (Trelstar)), LHRH antagonists (such as but not limited to Degarelix (Firmagon) and Relugolix (Orgovyx)), Abiraterone (Zytiga), Ketoconazole (Nizoral), First-generation anti-androgens (such as but not limited to Flutamide (Eulexin), Bicalutamide (Casodex), Nilutamide (Nilandron)) and second generation antiandrogens (such as but not limited to Enzalutamide (Xtandi), apalutamide (Erleada), and darolutamide (Nubeqa)).

Non limiting exmamples of chemotherapy are Docetaxel, Cabazitaxel, Mitoxantrone, Estramustine, and Carboplatin.

Non limiting examples of radiopharmaceuticals are Lutetium Lu 177 vipivotide tetraxetan (also known as 177Lu-PSMA-617 or Pluvicto), Radium-223 (Xofigo), Strontium-89 (Metastron), Samarium-153 (Quadramet).

Non limiting examples of immunotherapy are cancer vaccine (such as but not limited to Sipuleucel-T (Provenge) and prostatic acid phosphatase (PAP)), or Immune checkpoint inhibitors (such as but not limited to pembrolizumab (Keytruda) and dostarlimab (Jemperli)).

In a sixth aspect the invention relates to a kit of parts comprising a PDE4D7 specific antibody. The kit may optionally further comprise: a suitable secondary antibody that allows visualizing the primary antibody. Suitable secondary antibodies may be HRP or AP conjugated antibodies, or fluorescent dye conjugated antibody.

The antibody may be monoclonal or polyclonal. Preferably the antibody is directed to the unique N terminal sequence of PDE4D7. The N terminal sequence may be fused to a carrier protein such as GST as the unique N terminal sequence of PDE4D7 is short. Preferably the antibody is directed to SEQ ID NO: 3 or a part thereof or a sequence with at least 90% sequence homology to SEQ ID NO: 3.

SEQ ID NO: 3:

MKRNTCDLLSRSKSASEETLHSSNEEEDPFRGMEPYLVRRLSCRNIQLPPLAFRQLEQADLKSESE

NIQRPTSLPLKIL

It is commonly known how to raise monoclonal antibodies using e.g. hybridoma techniques (Lerner EA. How to make a hybridoma. Yale J Biol Med. 1981 Sep-Oct;54(5):387-402., incorporated by reference in its entirety) or polyclonal antibodies by immunization of an animal (The Laboratory Rabbit, Guinea Pig, Hamster, and Other Rodents

American College of Laboratory Animal Medicine, 2012, Pages 259-274 - Chapter 11 Polyclonal antibody production).

In a seventh aspect the invention relates to use of the kit of parts according to the sixth aspect of the invention quantifying PDE4D7 protein in the nuclei of cells in a sample, the method comprising: incubating the sample with the PDE4D7 specific antibody;

binding the stained sample with a suitable secondary antibody; detecting PDE4D7 by visualizing the secondary antibody; and detecting and optionally quantifying the nuclear localization of PDE4D7. In an embodiment the use further comprises a step of predicting an outcome for a patient with hormone sensitive prostate cancer based on the quantification. In an embodiment the use comprises performing the methods as defined in the first or second aspect of the invention.

In an embodiment the kit of parts is used to perform immunohistochemistry or immunofluorescence microscopy. In an embodiment the immunohistochemistry or immunofluorescence microscopy is performed on a section of a sample. In an embodiment the kit of parts is used to perform ELISA or Western blot on isolated cell nuclei from a sample.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

EXAMPLES

## Nuclear PDE4D7 expression in prostate cancer tissue

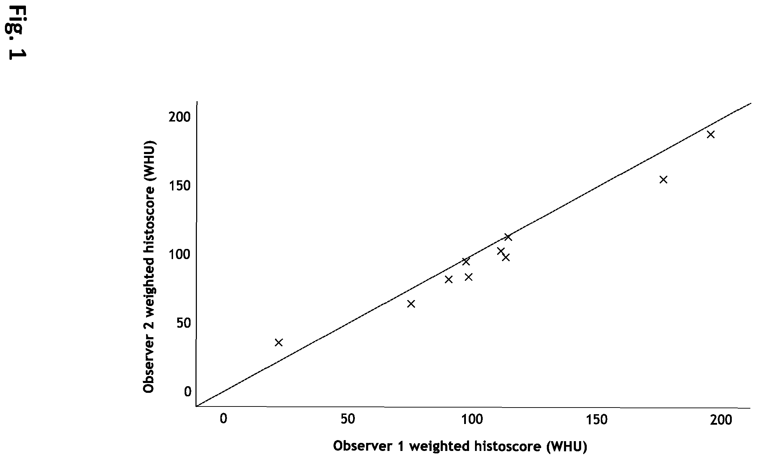

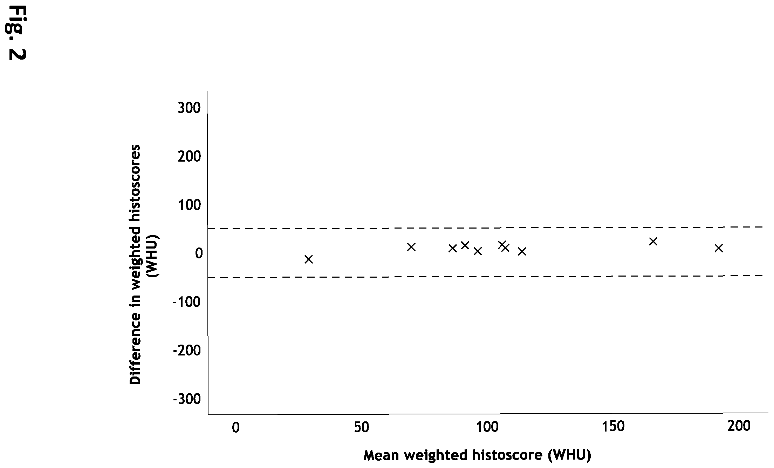

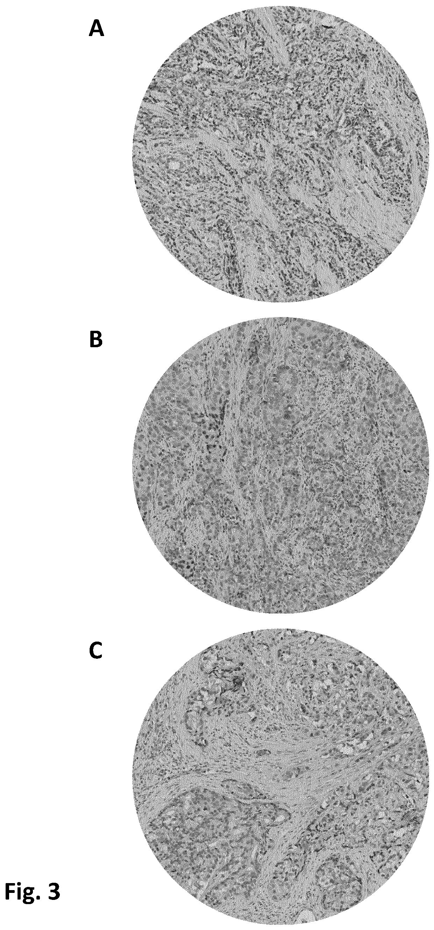

Nuclear PDE4D7 expression was analysed digitally using the open-source digital pathology platform QuPath and manually double-scored independently by Emma Parsons using full sections from 21 patients in the hormone-sensitive and castrate-resistant prostate cancer cohort. Five hormone-sensitive patients had tissue missing or contained no tumour leaving 16 patients for analysis. Two castrate-resistant patients had tissue missing or contained no tumour leaving 19 patients for analysis. The ICCC was 0.991, indicating an excellent correlation between independent observers (Fig. 1). Additionally, no score difference was $> \pm 50$ WHU (Fig. 2). Representative images of PDE4D7 staining pattern may be seen in Fig. 3.

## Nuclear PDE4D7 expression in hormone-sensitive tumours

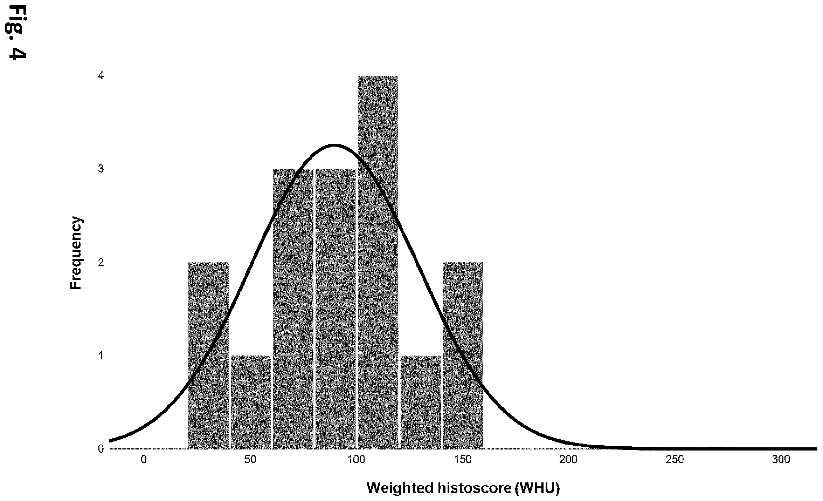

The WHS method was used to determine PDE4D7 expression in the nuclei of tumour cells (n=16). Hormone-sensitive nuclear PDE4D7 expression ranged from 22-155 WHU with a median of 90 WHU (IQR 64-112). The distribution of hormone-sensitive nuclear PDE4D7 expression is displayed in a histogram (Fig. 4). The median was used as the threshold (90 WHU); low expression was assigned a score of 0 and high expression was assigned a score of 1.

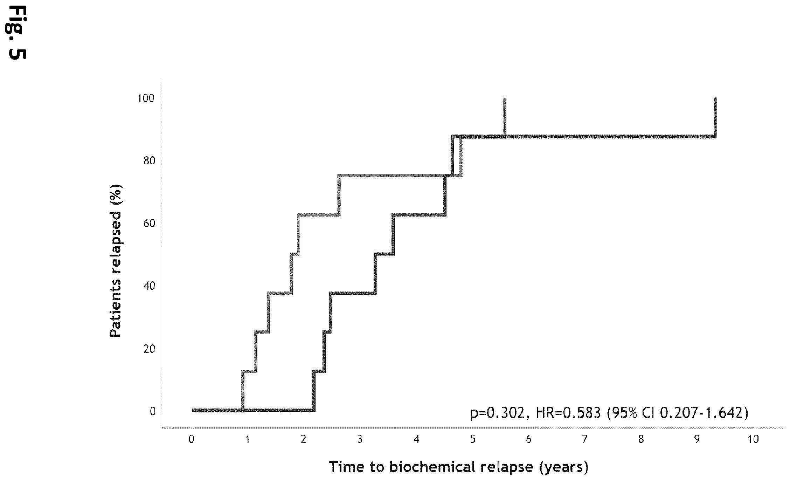

The relationship between hormone-sensitive nuclear PDE4D7 expression and both TTR (Time to Relapse) and CSS (Cancer Specific Survival) was determined using Kaplan-Meier survival curves. Low and high PDE4D7 expression was compared using the log rank test. Hormone-sensitive nuclear PDE4D7 expression was not associated with TTR (p=0.302, HR=0.583 (95% CI 0.207-1.642)) (Fig. 5).

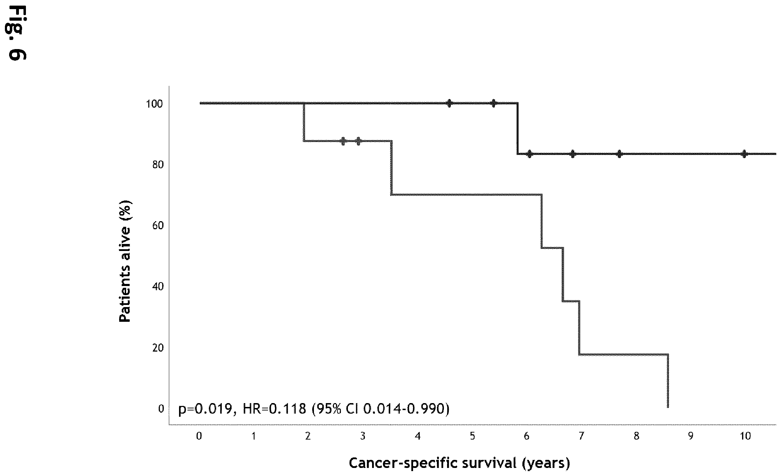

Hormone-sensitive nuclear PDE4D7 expression significantly associated with CSS (p=0.019, HR=0.118 (95% CI 0.014-0.990)) (Fig. 6). Low nuclear PDE4D7 expression significantly reduced CSS, stratifying mean survival from 10.05 years for patients with high expression to 5.83 years for patients with low expression. 5-year survival was reduced from 85% to 70% and 10-year survival was reduced from 85% to 0%.

To determine the association between hormone-sensitive nuclear PDE4D7 expression and clinico-pathological characteristics such as age, Gleason grade, serum PSA concentration and metastases at diagnosis, chi-squared analysis was performed. Nuclear PDE4D7 expression in hormone-sensitive tumours significantly associated with status ($\chi2(1)$ =6.904, p=0.009) (Table 1).

Table 1. Chi-squared table of associations between hormone-sensitive nuclear PDE4D7 expression and clinico-pathological characteristics of the hormone-sensitive and castrate-resistant prostate cancer cohort. n=number of patients; p=likelihood ratio chi-square p-value. Significant p-values are in bold.

| | Low, *n*=8 (%) | High, *n*=8 (%) | *p* |
|---|---|---|---|
| Age (years) at diagnosis | | | 1.000 |
| ≤70 | 4 (50.0) | 4 (50.0) | |
| >70 | 4 (50.0) | 4 (50.0) | |
| Gleason score at diagnosis | | | 0.614 |
| ≤7 | 4 (50.0) | 5 (62.5) | |
| >7 | 4 (50.0) | 3 (37.5) | |
| PSA level (ng/mL) at diagnosis | | | 0.519 |
| ≤10 | 1 (12.5) | 2 (25.0) | |
| >10 | 7 (87.5) | 6 (75.0) | |
| Metastases at diagnosis | | | 0.251 |
| Absent | 7 (87.5) | 7 (100.0) | |
| Present | 1 (12.5) | 0 (0.0) | |
| Status | | | 0.009 |

(continued)

| | Low, *n*=8 (%) | High, *n*=8 (%) | *p* |
|---|---|---|---|
| Alive | 0 (0.0) | 0 (0.0) | |
| Dead from cancer | 6 (75.0) | 1 (12.5) | |
| Dead from another cause | 2 (25.0) | 7 (87.5) | |

**Nuclear PDE4D7 expression in castrate-resistant tumours**

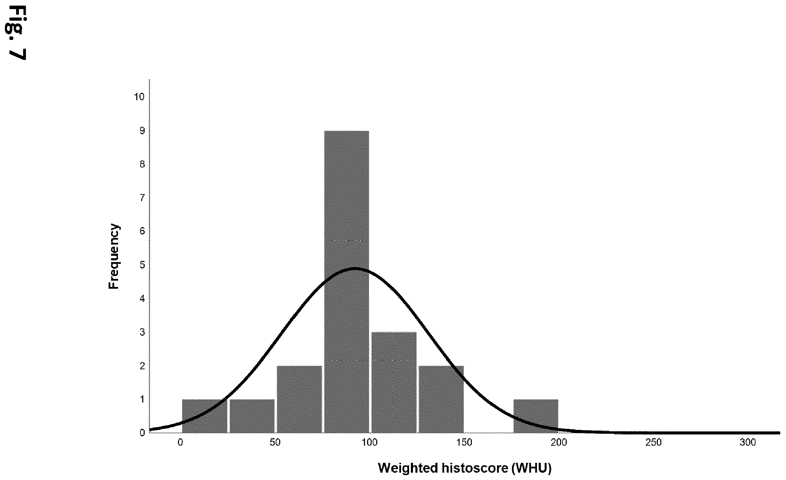

The WHS method was used to determine PDE4D7 expression in the nuclei of tumour cells (n=19). Castrate-resistant nuclear PDE4D7 expression ranged from 2-188 WHU with a median of 90 WHU (IQR 78-107). The distribution of castrate-resistant nuclear PDE4D7 expression is displayed in a histogram (Fig. 7). The median was used as the threshold (90 WHU); low expression was assigned a score of 0 and high expression was assigned a score of 1.

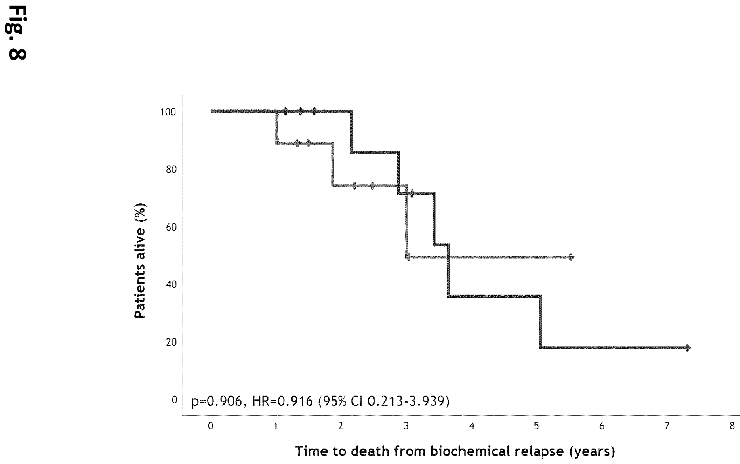

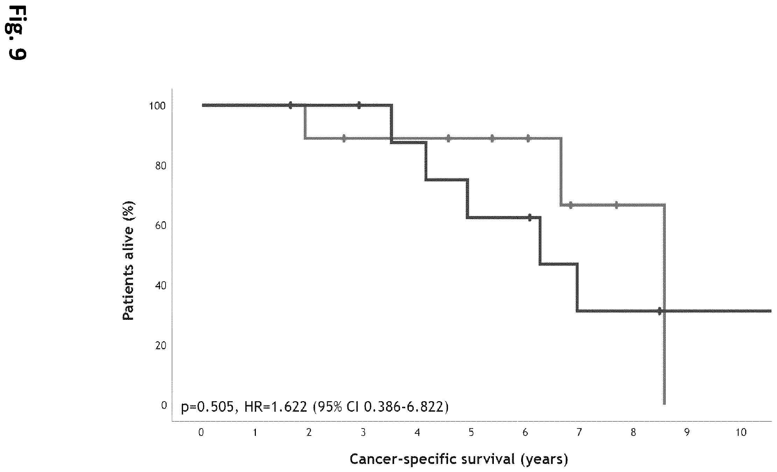

The relationship between castrate-resistant nuclear PDE4D7 expression and both TTDR (Time to Death from Relapse) and CSS was determined using Kaplan-Meier survival curves. Low and high expression was compared using the log rank test. Castrate-resistant nuclear PDE4D7 expression was not associated with TTDR (p=0.906, HR=0.916 (95% CI 0.213-3.939)) (Fig. 8). Castrate-resistant nuclear PDE4D7 expression was not associated with CSS (p=0.505, HR=1.622 (95% CI 0.386-6.822)) (Fig. 9).

To determine the association between castrate-resistant nuclear PDE4D7 expression and clinico-pathological characteristics such as Gleason Grade, serum PSA concentration, and metastases at relapse, chi-squared analysis was performed. Nuclear PDE4D7 expression in castrate-resistant tumours did not associate with any clinico-pathological characteristics in this cohort (Table 2).

*Table 2. Chi-squared table of associations between castrate-resistant nuclear PDE4D7 expression and clinico-pathological characteristics of the hormone-sensitive and castrate-resistant prostate cancer cohort. n=number of patients; p=likelihood ratio chi-square p-value.*

| | Low, *n*=9 (%) | High, *n*=10 (%) | *p* |
|---|---|---|---|
| Gleason score at relapse | | | 0.524 |
| ≤7 | 1 (11.1) | 2 (22.2) | |
| >7 | 8 (88.9) | 7 (77.8) | |
| Metastases at relapse | | | 0.548 |
| Absent | 1 (25.0) | 3 (42.9) | |
| Present | 3 (75.0) | 4 (57.1) | |
| Status | | | 0.461 |
| Alive | 0 (0) | 0 (0.0) | |
| Dead from cancer | 3 (33.3) | 5 (50.0) | |
| Dead from another cause | 6 (66.7) | 5 (50.0) | |
| PSA level (ng/mL) at relapse | | | 0.853 |
| ≤10 | 4 (44.4) | 2 (50.0) | |
| >10 | 5 (55.6) | 2 (50.0) | |

**Cytoplasmic PDE4D7 expression in prostate cancer tissue**

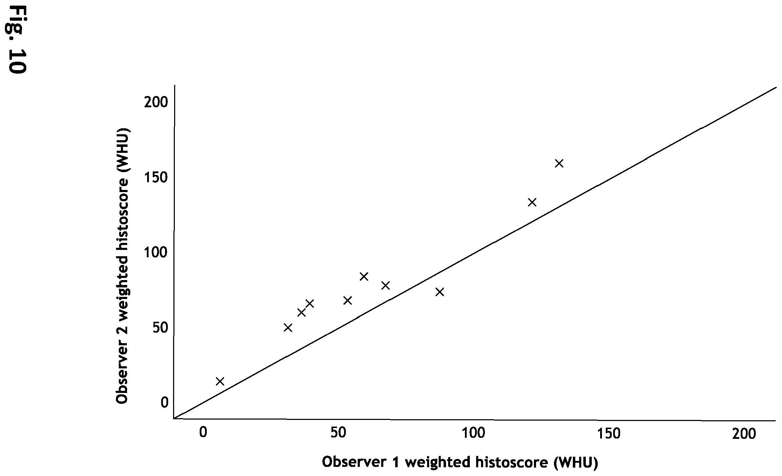

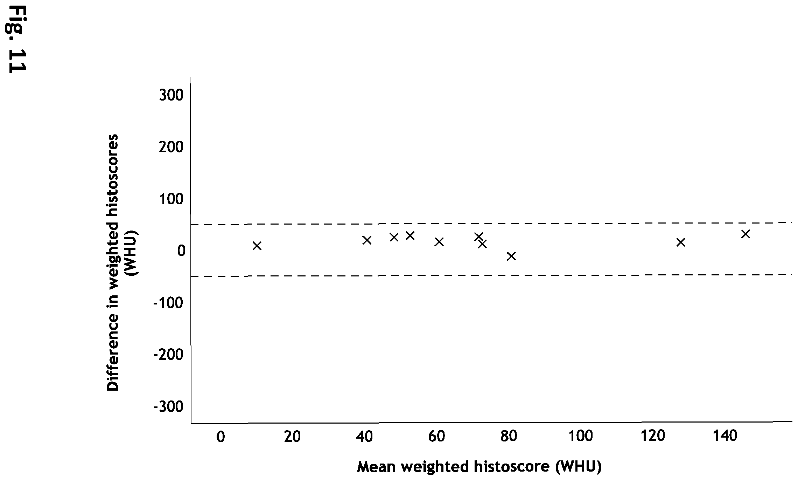

Cytoplasmic PDE4D7 expression was analysed digitally using the open-source digital pathology platform QuPath and manually double-scored independently by Emma Parsons using full sections from 21 patients in the hormone-sensitive and castrate-resistant prostate cancer cohort. Five hormone-sensitive patients had tissue missing or contained no tumour leaving 16 patients for analysis. Two castrate-resistant patients had tissue missing or contained no tumour leaving 19 patients for analysis. The ICC was 0.975, indicating an excellent correlation between independent observers (Fig. 10). Additionally, no score bias or difference was >±50 WHU (Fig. 11).

**Cytoplasmic PDE4D7 expression in hormone-sensitive tumours**

The WHS method was used to determine PDE4D7 expression in the cytoplasm of tumour cells (n=16). Hormone-sensitive cytoplasmic PDE4D7 expression ranged from 4-131 WHU with a median of 53 WHU (IQR 35-77). The distribution of cytoplasmic PDE4D7 expression is displayed in a histogram (Fig. 6.12). The median was used as the threshold (53 WHU); low expression was assigned a score of 0 and high expression was assigned a score of 1.

The relationship between hormone-sensitive cytoplasmic PDE4D7 expression and both TTR and CSS was determined using Kaplan-Meier survival curves. Low and high PDE4D7 expression was compared using the log rank test. Hormone-sensitive cytoplasmic PDE4D7 expression was not associated with TTR (p=0.448, HR=0.672 (95% CI 0.239-1.888)) (Fig. 6.13). Hormone-sensitive cytoplasmic PDE4D7 expression was not associated with CSS (p=0.129, HR=0.300 (95% CI 0.058-1.558)) (Fig. 6.14).

To determine the association between hormone-sensitive cytoplasmic PDE4D7 expression and clinico-pathological characteristics such as age, Gleason grade, serum PSA concentration, and metastases at diagnosis, chi-squared analysis was performed. Cytoplasmic PDE4D7 expression in hormone-sensitive tumours did not associate with any clinico-pathological characteristics in this cohort (Table 3).

*Table 3. Chi-squared table of associations between hormone-sensitive cytoplasmic PDE4D7 expression and clinico-pathological characteristics of the hormone-sensitive and castrate-resistant prostate cancer cohort. n=number of patients; p=likelihood ratio chi-square p-value.*

|  | Low, *n*=8 (%) | High, *n*=8 (%) | *p* |
|---|---|---|---|
| Age (years) at diagnosis |  |  | 1.000 |
| ≤70 | 4 (50.0) | 4 (50.0) |  |
| >70 | 4 (50.0) | 4 (50.0) |  |
| Gleason score at diagnosis |  |  | 0.614 |
| ≤7 | 4 (50.0) | 5 (62.5) |  |
| >7 | 4 (50.0) | 3 (37.5) |  |
| Metastases at diagnosis |  |  | 0.205 |
| Absent | 8 (100.0) | 6 (85.7) |  |
| Present | 0 (0.0) | 1 (14.3) |  |
| Status |  |  | 0.125 |
| Alive | 0 (0.0) | 0 (0.0) |  |
| Dead from cancer | 5 (62.5) | 2 (25.0) |  |
| Dead from another cause | 3 (37.5) | 6 (75.0) |  |
| PSA level (ng/mL) at diagnosis |  |  | 0.519 |
| ≤10 | 1 (12.5) | 2 (25.0) |  |
| >10 | 7 (87.5) | 6 (75.0) |  |

**Cytoplasmic PDE4D7 expression in castrate-resistant tumours**

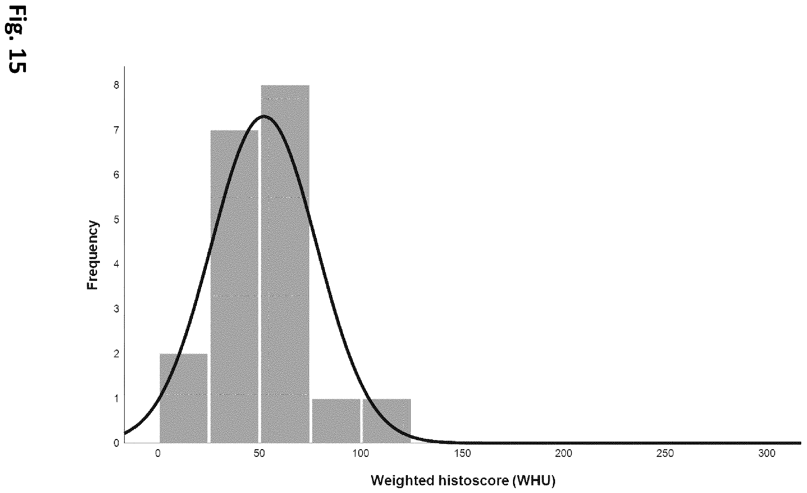

The WHS method was used to determine PDE4D7 expression in the cytoplasm of tumour cells (n=19). Castrate-resistant cytoplasmic PDE4D7 expression ranged from 1-121 WHU with a median of 53 WHU (IQR 41-60). The distribution of castrate-resistant cytoplasmic PDE4D7 expression is displayed in a histogram (Fig. 15). The median was used as the threshold (53 WHU); low expression was assigned a score of 0 and high expression was assigned a score of 1.

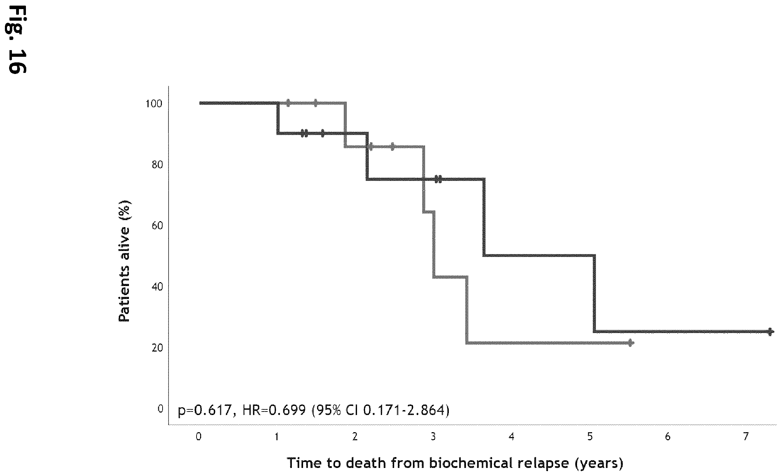

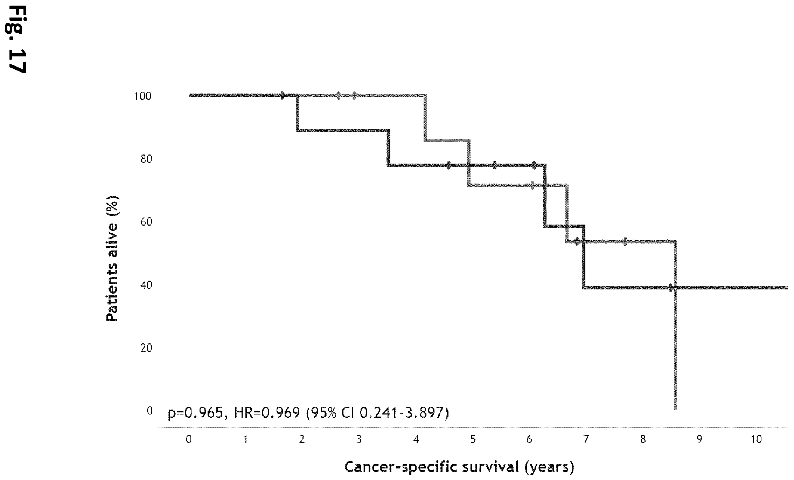

The relationship between castrate-resistant cytoplasmic PDE4D7 expression and both TTDR and CSS was determined using Kaplan-Meier survival curves. Low and high PDE4D7 expression was compared using the log rank test. Castrate-resistant cytoplasmic PDE4D7 expression was not associated with TTDR (p=0.617, HR=0.699 (95% CI 0.171-2.864)) (Fig. 16). Castrate-resistant cytoplasmic PDE4D7 expression was not associated with CSS (p=0.965, HR=0.969 (95% CI 0.241-3.897)) (Fig. 17).

To determine the association between castrate-resistant cytoplasmic PDE4D7 expression and clinico-pathological characteristics such as Gleason Grade, serum PSA concentration, and metastases at relapse, chi-squared analysis was performed. Cytoplasmic PDE4D7 expression in castrate-resistant tumours did not associate with any clinico-pathological characteristics in this cohort (Table 4).

*Table 4. Chi-squared table of associations between castrate-resistant cytoplasmic PDE4D7 expression and clinico-pathological characteristics of the hormone-sensitive and castrate-resistant prostate cancer cohort. n=number of patients; p=likelihood ratio chi-square p-value.*

|  | Low, *n*=9 (%) | High, *n*=10 (%) | *p* |
|---|---|---|---|
| Gleason score at relapse |  |  | 0.524 |
| ≤7 | 1 (11.1) | 2 (22.2) |  |

(continued)

|  | Low, *n*=9 (%) | High, *n*=10 (%) | *p* |
|---|---|---|---|
| >7 | 8 (88.9) | 7 (77.8) | |
| PSA level (ng/mL) at relapse | | | 0.797 |
| ≤10 | 3 (42.9) | 3 (50.0) | |
| >10 | 4 (57.1) | 3 (50.0) | |
| Metastases at relapse | | | 0.819 |
| Absent | 2 (33.3) | 2 (40.0) | |
| Present | 4 (66.7) | 3 (60.0) | |
| Status | | | 0.845 |
| Alive | 0 (0.0) | 0 (0.0) | |
| Dead from cancer | 4 (44.4) | 4 (40.0) | |
| Dead from another cause | 5 (55.6) | 6 (60.0) | |

**Membranous PDE4D7 expression in prostate cancer tissue**

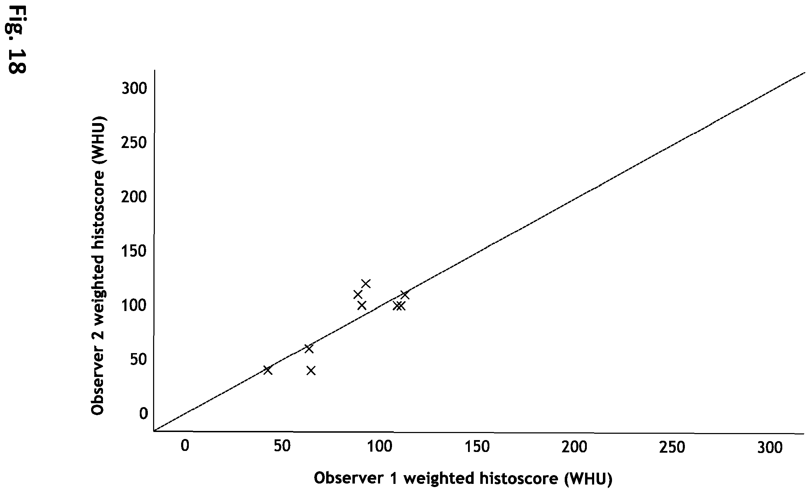

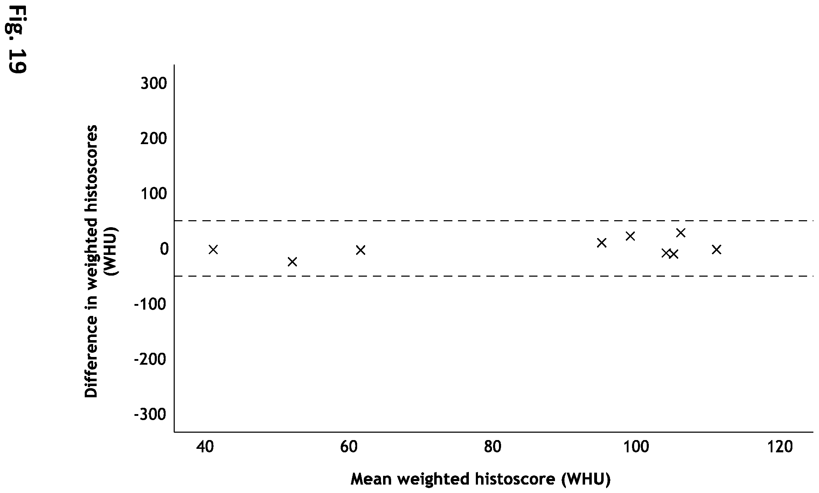

Due to the challenges faced with the open-source digital pathology platform QuPath not being able to accurately assess membrane expression levels, membranous PDE4D7 expression was analysed manually and double-scored independently by a second investigator using full sections from 21 patients in the hormone-sensitive and castrate-resistant prostate cancer cohort. Five hormone-sensitive patients had tissue missing or contained no tumour leaving 16 patients for analysis. Two castrate-resistant patients had tissue missing or contained no tumour leaving 19 patients for analysis. The ICCC was 0.938, indicating an excellent correlation between independent observers (Fig. 18). Additionally, no score difference was >±50 WHU (Fig. 19).

**Membranous PDE4D7 expression in hormone-sensitive tumours**

The WHS method was used to determine PDE4D7 expression on the membrane of tumour cells (n=16). Hormone-sensitive membranous PDE4D7 expression ranged from 22-116 WHU with a median of 79 WHU (IQR 65-91.5). The distribution of hormone-sensitive membranous PDE4D7 expression is displayed in a histogram (Fig. 6.20). The median was used as the threshold (79 WHU); low expression was assigned a score of 0 and high expression was assigned a score of 1.

The relationship between hormone-sensitive membranous PDE4D7 expression and both TTR and CSS was determined using Kaplan-Meier survival curves. Low and high PDE4D7 expression was compared using the log rank test. Hormone-sensitive membranous PDE4D7 expression was not associated with TTR (p=0.315, HR=0.592 (95% CI 0.211-1.664)) (Fig. 6.21). Hormone-sensitive membranous PDE4D7 expression was not associated with CSS (p=0.801, HR=0.824 (95% CI 0.182-3.727)) (Fig. 6.22).

To determine the association between hormone-sensitive membranous PDE4D7 expression and clinico-pathological characteristics such as age, Gleason grade, serum PSA concentration, metastases at diagnosis and status, chi-squared analysis was performed. Membranous PDE4D7 expression in hormone-sensitive tumours did not associate with any clinico-pathological characteristics in this cohort (Table 5).

*Table 5. Chi-squared table of associations between hormone-sensitive membranous PDE4D7 expression and clinico-pathological characteristics of the hormone-sensitive and castrate-resistant prostate cancer cohort. n=number of patients; p=likelihood ratio chi-square p-value.*

|  | Low, *n*=8 (%) | High, *n*=8 (%) | *p* |
|---|---|---|---|
| Age (years) at diagnosis | | | 0.315 |
| ≤70 | 5 (62.5) | 3 (37.5) | |
| >70 | 3 (37.5) | 5 (62.5 | |
| Gleason score at diagnosis | | | 0.614 |
| ≤7 | 5 (62.5) | 4 (50.0) | |
| >7 | 4 (37.5) | 4 (50.0) | |
| PSA level (ng/mL) at diagnosis | | | 0.519 |
| ≤10 | 2 (25.0) | 1 (12.5) | |
| >10 | 6 (75.0) | 7 (87.5) | |

(continued)

| | Low, *n*=8 (%) | High, *n*=8 (%) | *p* |
|---|---|---|---|
| Metastases at diagnosis | | | 0.251 |
| Absent | 7 (87.5) | 7 (100.0) | |
| Present | 1 (12.5) | 0 (0.0) | |
| Status | | | 0.614 |
| Alive | 0 (0.0) | 0 (0.0) | |
| Dead from cancer | 3 (37.5) | 4 (50.0) | |
| Dead from another cause | 5 (62.5) | (50.0) | |

**Membranous PDE4D7 expression in castrate-resistant tumours**

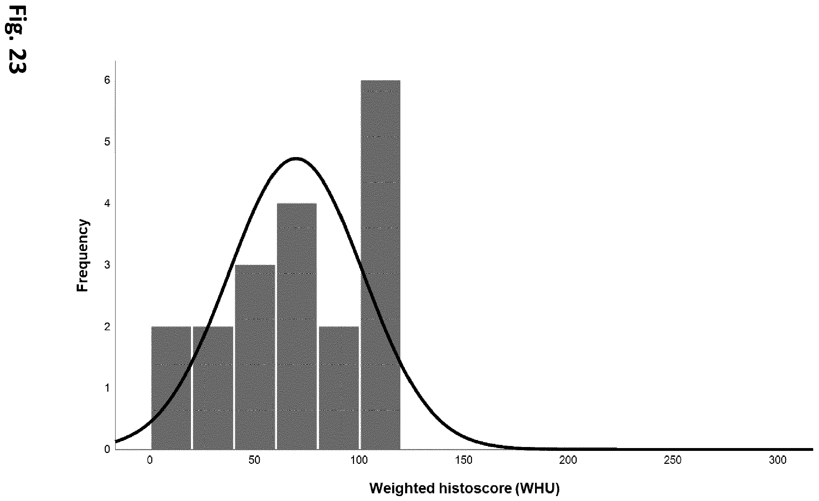

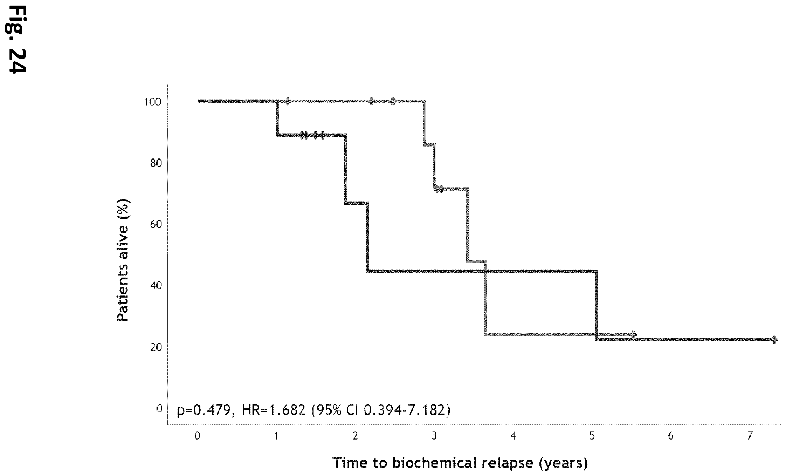

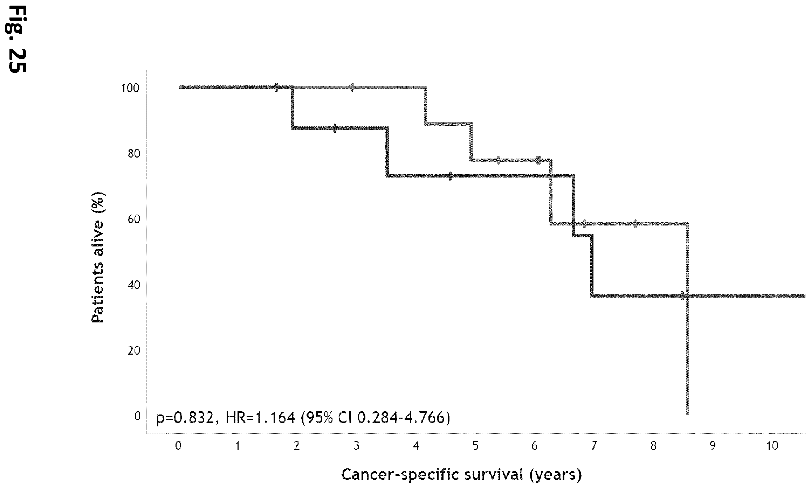

The WHS method was used to determine PDE4D7 expression in the membrane of tumour cells (n=19). Castrate-resistant membranous PDE4D7 expression ranged from 14-110 WHU with a median of 72 WHU (IQR 40-100). The distribution of castrate-resistant membranous PDE4D7 expression is displayed in a histogram (Fig. 23). The median was used as the threshold (72 WHU); low expression was assigned a score of 0 and high expression was assigned a score of 1.
The relationship between castrate-resistant membranous PDE4D7 expression and both TTDR and CSS was determined using Kaplan-Meier survival curves. Low and high PDE4D7 expression was compared using the log rank test. Castrate-resistant membranous PDE4D7 expression was not associated with TTDR (p=0.479, HR=1.682 (95% CI 0.394-7.182)) (Fig. 24). Castrate-resistant membranous PDE4D7 expression was not associated with CSS (p=0.832, HR=1.164 (95% CI 0.284-4.766)) (Fig. 25).
To determine the association between castrate-resistant membranous PDE4D7 expression and clinico-pathological characteristics such as Gleason Grade, serum PSA concentration, and metastases at relapse, chi-squared analysis was performed. Membranous PDE4D7 expression in castrate-resistant tumours significantly associated with Gleason score at relapse ($\chi^2(1)=5.635$, p=0.018) (Table 6).

*Table 6. Chi-squared table of associations between castrate-resistant membranous PDE4D7 expression and clinico-pathological characteristics of the hormone-sensitive and castrate-resistant prostate cancer cohort. n=number of patients; p=likelihood ratio chi-square p-value. Significant p-values are in bold.*

| | Low, *n*=10 (%) | High, *n*=9 (%) | *p* |
|---|---|---|---|
| Gleason score at relapse | | | **0.018** |
| ≤7 | 0 (0.0) | 3 (37.5) | |
| >7 | 10 (100.0) | 5 (62.5) | |
| PSA level (ng/mL) at relapse | | | **0.388** |
| ≤10 | 4 (57.1) | 2 (33.3) | |
| >10 | 3 (42.9) | 4 (66.7) | |
| Metastases at relapse | | | **0.819** |
| Absent | 2 (33.3) | 2 (40.0) | |
| Present | 4 (66.7) | 3 (60.0) | |
| Status | | | **0.258** |
| Alive | 0 (0.0) | 0 (0.0) | |
| Dead from cancer | 4 (40.0) | 4 (44.4) | |
| Dead from another cause | 6 (60.0) | 5 (55.6) | |

**Methods**

**Tissue Studies for PDE4D7 IHC (Immunohistochemistry)**

Before this research, FFPE prostate cancer blocks were obtained from NHS pathology archives. Full sections from each block underwent haematoxylin and eosin (H&E) staining, identifying tumor-rich areas. Tissue from 21 patients with matched hormone-sensitive and castrate-resistant tumor pairs, initially diagnosed between 1984 and 2000, underwent immunohistochemistry (IHC). An anonymized database linked clinical information to the tissue. Patient selection relied on initial hormone treatment response and subsequent biochemical relapse (two PSA rises >10%). Hormone-sensitive tissue came from transrectal ultrasound (TRUS-) guided biopsies or transurethral resection of the prostate (TURP). Castrate-resistant tissue came from TURP to relieve bladder outflow obstruction. All tissue was FFPE, with a full PSA profile for each patient.

**Tissue Preparation**

The FFPE tissue blocks were cut into 2.5 $\mu$m thick sections, which were then baked and stored.

**Dewaxing and Rehydrating Tissue Sections**

Full sections were dewaxed in Histo-Clear (National Diagnostics, Atlanta) (3 minutes twice) and rehydrated through a series of graded alcohols (100% alcohol for 3 minutes, twice; 90% alcohol for 2 minutes; 70% alcohol for 2 minutes) before being washed in running water for 5 minutes.

**Antigen Retrieval**

Heat-mediated antigen retrieval was performed using Tris-EDTA (TE) Buffer pH 8.0, heated to boiling for 14 minutes in the microwave, before sections were added and heated under pressure for 5 minutes. The buffer and slides were left to cool for 30 minutes.

**Blocking**

Slides were incubated for 10 minutes in 3% hydrogen peroxide and washed in water. This step prevents 3,3'-Diaminobenzidine (DAB) from reacting with endogenous peroxidases. Slides were then incubated for 30 minutes at room temperature in 1.5% horse serum (Vector Laboratories Inc., Newark, California) diluted in antibody diluent (Agilent Dako, Santa Clarita, California). This step prevents nonspecific background staining between the primary antibody and other tissue proteins.

**Incubation with Primary Antibody**

Sections incubated in primary antibody (diluted in antibody diluent; Table 7). Optimal dilutions were determined using needle biopsy prostate cancer tissue.

**Incubation with Secondary Antibody**

Sections were washed twice in TBS for 5 minutes before being incubated for 30 minutes at room temperature in ImmPRESS® HRP Universal Antibody Polymer Detection Kit, Peroxidase (Vector Laboratories, Newark, California) which contained secondary antibodies against the appropriate immunoglobulins in the primary antibody (Table 8). Sections were washed twice in TBS for 5 minutes.

**Antibody Detection and Counterstaining**

Detection of the secondary antibody was achieved using the DAB peroxidase substrate kit (Vector Laboratories, Newark, California). One drop of DAB substrate was diluted in 1 mL of DAB diluent. Sections were incubated in DAB substrate for 5 minutes at room temperature in the dark. Sections were washed in running water for 10 minutes. Counterstaining was performed to enhance visualisation of stained antigens by contrasting the colour of unstained tissue. Sections were incubated in haematoxylin for 1 minute and rinsed in running water for 2 minutes. Excess haematoxylin was removed by dipping sections in 0.5% acid alcohol (hydrochloric acid in ethanol) for 5 seconds and sections were rinsed in running water for 2 minutes. Sections were incubated in Scott's tap water for 2 minutes and rinsed in running water for 2 minutes.

**Dehydrating and Mounting Tissue Sections**

Sections were dehydrated through a series of graded alcohols (70% alcohol for 1 minute; 90% alcohol for 1 minute; 100% alcohol for 1 minute, twice) before being incubated in Histo-Clear for 1 minute, twice. Sections were mounted onto glass coverslips using Pertex® Mounting Medium (Histolab Products AB, Gothenburg, Sweden).

**Data Analysis**

PDE4D7 membrane expression were scored manually with 25% manual scoring by an independent second observer employing the weighted histoscore (WHS) method. The WHS method combines staining intensity with the percentage of positive cells. WHS assessment was performed at 20X magnification using
Equation **1**, giving a range of scores between 0 and 300 weighted histoscore units (WHU). Membrane histoscores were determined by allocating negative staining a 0, weak staining a 1, moderate staining a 2 and strong staining a 3.

$$0 \times (\% \ cells \ not \ stained) + 1 \times (\% \ cells \ weakly \ stained) + 2 \times (\% \ cells \ moderately \ stained)$$
$$+ 3 \times (\% \ cells \ strongly \ stained)$$

**Equation 1. Weighted histoscore assessment.**

PDE4D7 nuclear and cytoplasmic expression were scored using QuPath version 0.4.3 (Bankhead et al., 2017) with 25% manual scoring for validation as described above. QuPath is a software used for digital pathology analysis featuring algorithms for cell detection and interactive machine learning for object classification. To train the object classifier (Random trees), a training subset of around three images was used. A small circular annotation containing both tumour and stroma was created on each image, ensuring any areas of folded tissue or out of focus tissue was removed from the ROI. A preprocessing step was performed to increase the quality of the staining using automatic estimated stain vectors. Watershed cell detection was performed followed by training the object classifier by annotating a small region of tumour and of stroma. Intensity thresholds were set by manually identifying a cell classified as weakly, moderately and strongly stained. The training classifier was saved and used for all images for the same protein to ensure consistency. Five ROIs were annotated on each of the images in the full set and the training classifier was applied. The "tumor H-score" (Histoscore) was exported for each image. Individual classifiers were used for nuclear and cytoplasmic staining for each protein.

All statistical analyses were performed using IBM® SPSS® Statistics software version 28.0.0.0 (190) (IBM, Armonk, New York). Observer scores were compared by calculating the intraclass correlation coefficient (ICC) with an ICC of 0.8 indicating similarity of scores to be acceptable. A scatter plot to assess correlation and Bland Altman plot to assess for bias were created for the scores and the difference between each score between observers was confirmed to be $< \pm 50$ WHU. Due to low patient numbers, the median value was used to set the threshold for high and low expression of each protein. Kaplan-Meier log rank curves were used to identify associations between protein expression and time to biochemical relapse (TTR), time to death from biochemical relapse (TTDR) and cancer-specific survival (CSS). Cox regression was performed to determine hazard ratios. Chi-squared testing was performed to assess association between expression of each protein and several clinicopathological characteristics. Statistical significance was set to *p<0.05.*

Table 7. Primary Antibodies used for Immunohistochemistry.

| Antigen | Antigen retrieval | Clone | Isotype | Supplier | Cat # | Species | Dilution | Blocking | Incubation |
|---|---|---|---|---|---|---|---|---|---|
| DHX9 (RNA helicase A) | Tris-EDTA, pH 8.0 | N/A | IgG | Abcam | ab26271 | Rabbit | 1:2500 | 1.5% horse serum | 4 °C, overnight |
| PDE4D5 | Tris-EDTA, pH 8.0 | N/A | Unknown | In-house | N/A | Rabbit | 1:3000 | 1.5% horse serum | 4 °C, overnight |
| PDE4D7 | Tris-EDTA, pH 8.0 | N/A | Unknown | In-house | N/A | Sheep | 1:500 | 1.5% horse serum | Room temperature (20-25 °C), 3 h |
| PSMA | Tris-EDTA, pH 8.0 | IH8H5 | IgG1 | Invitrogen | 37-3900 | Mouse | 1:150 | 1.5% horse serum | 4 °C, overnight |

Table 8. Secondary Antibody HRP Detection Kits used for Immunohistochemistry.

| Antigen | Reactivity | Supplier | Cat # | Species |
|---|---|---|---|---|
| Anti-Mouse/ Rabbit IgG | Mouse and rabbit IgG primary antibodies. | Vector Laboratories | MP-7500 | Horse |
| Anti-Goat IgG | Goat, sheep, and bovine IgG primary antibodies. | Vector Laboratories | MP-7405 | Horse |

**Claims**

1. A computer implemented method for predicting an outcome for a patient with hormone sensitive prostate cancer, the method comprising:

   receiving a quantification of PDE4D7 protein in the nuclei of cells in a sample obtained from the patient; wherein the quantification is low or high nuclear presence of PDE4D7, and wherein low nuclear presence of PDE4D7 indicates a poor expected outcome and high nuclear presence of PDE4D7 status indicates a good expected outcome.

2. A method for predicting an outcome for a patient with hormone sensitive prostate cancer, the method comprising:

   performing a quantification of PDE4D7 protein in the nuclei of cells in a sample obtained from the patient; wherein the quantification is low or high nuclear presence of PDE4D7, and wherein low nuclear presence of PDE4D7 indicates a poor expected outcome and high nuclear presence of PDE4D7 status indicates a good expected outcome.

3. Method according to claim 2 wherein the quantification of PDE4D7 protein in the nuclei of cells is performed using an antibody based technology or using a mass spectrometry based technology, preferably wherein the quantification of PDE4D7 protein in the nuclei of cells is performed by immunohistochemistry staining or immunofluorescence staining of the sample, ELISA, Western blot or mass spectrometry analysis on isolated nuclei from the sample, or by performing mass spectrometry imaging on the sample.

4. Method according to any one of the preceding claims wherein the quantification is determined by calculating a weighted histoscore for nuclear localization of PDE4D7 in the nuclei of the sample.

5. Method according to claim 4 wherein the weighted histoscore is calculated using the formula:
   $0\times$(% cells not stained)$+1\times$(% cells weakly stained in the nucleus)$+2\times$(% cells moderately stained in the nucleus)$+3\times$(% cells strongly stained in the nucleus).

6. Method according to claims 4 or 5 wherein low or high nuclear presence of PDE4D7 protein in the nuclei of cells in the sample is determined when the weighted histoscore is below or above the mean reference value, wherein the mean reference value is predetermined in a cohort of reference samples, preferably prostate cancer reference samples, more preferably hormone sensitive prostate cancer reference samples.

7. Method according to claim 4 or 5 wherein low or high nuclear presence of PDE4D7 protein in the nuclei of cells in the sample is determined when the weighted histoscore is below or above a preset reference value, preferably wherein the reference value is predetermined in a cohort of reference samples, preferably prostate cancer reference samples, more preferably hormone sensitive prostate cancer reference samples, preferably wherein the preset value is a weighted histoscore of 90.

8. Method according to any one of the preceding claims wherein the sample is a prostate cancer sample or a metastasis of a prostate tumor.

9. Method according to any one of the preceding claims wherein the outcome is cancer specific survival.

10. A computer program product comprising instructions which when the program is executed by a computer cause the computer to carry out a method for predicting an outcome for a patient with hormone sensitive prostate cancer comprising the steps as defined in any one of claims 1 to 9.

11. An apparatus for predicting an outcome for a patient with hormone sensitive prostate cancer, comprising:

- an input adapted to receive a quantification of PDE4D7 protein in the nuclei of cells in a sample obtained from the patient;
- a processor adapted to determine the prediction of outcome based on the quantification of PDE4D7 protein in the nuclei of cells in a sample, and
- optionally, a providing unit adapted to provide the prediction to a medical caregiver or the subject.

12. A therapy for use in the treatment, alleviation or prevention of hormone sensitive prostate cancer in a patient, the use comprising predicting an outcome for the patient based on a quantification of PDE4D7 protein in the nuclei of cells in a sample obtained from the patient, and administering the therapy to the patient based on the outcome,

wherein when the outcome is favorable the therapy is a monotherapy selected from surgery, radiation therapy, hormonal treatment or chemotherapy, and
wherein when the outcome is not favorable the therapy is a combination therapy comprising at least one therapy selected from surgery, radiation therapy, hormonal treatment, chemotherapy, immunotherapy or radiopharmaceutical based therapy;
wherein the patient is a patient with hormone sensitive prostate cancer.

13. A kit of parts comprising a PDE4D7 specific antibody.

14. Use of the kit of parts according to claim 13 in quantifying PDE4D7 protein in the nuclei of cells in a sample, the method comprising:

incubating the sample with the PDE4D7 specific antibody;
binding the stained sample with a suitable secondary antibody;
detecting PDE4D7 by visualizing the secondary antibody; and
and detecting and optionally quantifying the nuclear localization of PDE4D7.

15. Use according to claim 14 wherein the use further comprises a step of predicting an outcome for a patient with hormone sensitive prostate cancer based on the quantification.

Fig. 1

Fig. 2

A

B

C

**Fig. 3**

Fig. 4

**Fig. 5**

p=0.302, HR=0.583 (95% CI 0.207-1.642)

Patients relapsed (%)

Time to biochemical relapse (years)

Fig. 6

Fig. 7

**Fig. 8**

Fig. 9

**Fig. 10**

**Fig. 11**

Difference in weighted histoscores (WHU)

Mean weighted histoscore (WHU)

EP 4 603 836 A1

**Fig. 12**

**Fig. 13**

Fig. 14

Fig. 15

EP 4 603 836 A1

Weighted histoscore (WHU)

**Fig. 16**

**Fig. 17**

Fig. 18

EP 4 603 836 A1

Fig. 19

Fig. 20

**Fig. 21**

Fig. 22

Fig. 23

EP 4 603 836 A1

Weighted histoscore (WHU)

Fig. 24

Fig. 25

## EUROPEAN SEARCH REPORT

**Application Number**

EP 24 15 7256

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/131194 A1 (KONINKL PHILIPS ELECTRONICS NV [NL]; UNIV GLASGOW [GB] ET AL.) 18 November 2010 (2010-11-18) | 12,13 | INV.<br>G01N33/574 |
| A | * the whole document *<br>* abstract *<br>* page 1, paragraph 1 * | 1-11,14, 15 | |
| | ----- | | |
| X | CHLOE GULLIVER: "and is implicated in prostate cancer progression",<br>MOLECULAR ONCOLOGY,<br>vol. 18, no. 3,<br>5 January 2024 (2024-01-05), pages 707-725, XP93167970,<br>ISSN: 1574-7891, DOI:<br>10.1002/1878-0261.13572<br>Retrieved from the Internet:<br>URL:https://www.ncbi.nlm.nih.gov/pmc/artic les/PMC10920091/pdf/MOL2-18-707.pdf> | 12-14 | |
| A | * the whole document *<br>* abstract *<br>* figure 1C *<br>* section 2.7 * | 1-11,15 | |
| | ----- | | TECHNICAL FIELDS SEARCHED (IPC)<br><br>G01N |
| X | RENÉ BÖTTCHER ET AL: "Human PDE4D isoform composition is deregulated in primary prostate cancer and indicative for disease progression and development of distant metastases",<br>ONCOTARGET,<br>vol. 7, no. 43,<br>25 October 2016 (2016-10-25), pages 70669-70684, XP055505101,<br>United States<br>ISSN: 1949-2553, DOI:<br>10.18632/oncotarget.12204 | 12 | |
| A | * the whole document *<br>* abstract * | 1-11, 13-15 | |
| | ----- | | |

The present search report has been drawn up for all claims

| Place of search | Date of completion of the search | Examiner |
|---|---|---|
| The Hague | 9 July 2024 | Jenkins, Gareth |

**ANNEX TO THE EUROPEAN SEARCH REPORT
ON EUROPEAN PATENT APPLICATION NO.**     EP 24 15 7256

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2024

| Patent document cited in search report | Publication date | Patent family member(s) | | Publication date |
|---|---|---|---|---|
| WO 2010131194 A1 | 18-11-2010 | BR | PI1007704 A2 | 03-12-2019 |
| | | CN | 102439176 A | 02-05-2012 |
| | | DK | 2430190 T3 | 18-02-2019 |
| | | EP | 2430190 A1 | 21-03-2012 |
| | | ES | 2707598 T3 | 04-04-2019 |
| | | JP | 6246167 B2 | 13-12-2017 |
| | | JP | 6588496 B2 | 09-10-2019 |
| | | JP | 2012526544 A | 01-11-2012 |
| | | JP | 2016028245 A | 25-02-2016 |
| | | JP | 2017212973 A | 07-12-2017 |
| | | JP | 2019205431 A | 05-12-2019 |
| | | KR | 20120036842 A | 18-04-2012 |
| | | KR | 20180002900 A | 08-01-2018 |
| | | RU | 2011150264 A | 20-06-2013 |
| | | US | 2012129788 A1 | 24-05-2012 |
| | | WO | 2010131194 A1 | 18-11-2010 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BÖTTCHER et al.** *Br J Cancer.*, 17 November 2015, vol. 113 (10), 1502-11
- **GULLIVER et al.** Loss of PDE4D7 expression promotes androgen independence, neuroendocrine differentiation and alterations in DNA repair: implications for therapeutic strategies.. *Br J Cancer.*, October 2023, vol. 129 (9), 1462-1476
- **RIZZARDI et al.** *Diagn Pathol*, 2012, vol. 7, 42
- **YALE.** *J Biol Med.*, September 1981, vol. 54 (5), 387-402
- College of Laboratory Animal Medicine. 2012, 259-274